(19) Europäisches Patentamt
European Patent Office
Office européen des brevets

(11) **EP 4 366 674 B1**

(12) **EUROPEAN PATENT SPECIFICATION**

(45) Date of publication and mention
of the grant of the patent:
**14.01.2026 Bulletin 2026/03**

(21) Application number: **22748647.9**

(22) Date of filing: **07.07.2022**

(51) International Patent Classification (IPC):
***A61F 9/00*** *(2006.01)*

(52) Cooperative Patent Classification (CPC):
**A61F 9/0017**

(86) International application number:
**PCT/US2022/036418**

(87) International publication number:
**WO 2023/283379 (12.01.2023 Gazette 2023/02)**

(54) **APPARATUS FOR DELIVERY OF OCULAR IMPLANTS**

VORRICHTUNG ZUR FREISETZUNG VON AUGENIMPLANTATEN

APPAREIL DE MISE EN PLACE D'IMPLANTS OCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.07.2021 US 202163219480 P**

(43) Date of publication of application:
**15.05.2024 Bulletin 2024/20**

(73) Proprietor: **Allergan, Inc.**
**North Chicago, IL 60064 (US)**

(72) Inventors:
• **GHEBREMESKEL, Alazar**
**Irvine, California 92612 (US)**
• **ROBINSON, Michael**
**Huntington Beach, California 92606 (US)**

• **NAGAR, Saumya**
**San Diego, California 92126 (US)**
• **NOVAKOVIC, Zoran**
**Irvine, California 92614 (US)**
• **ISIDORO, Michael**
**Newport Beach, California 92663 (US)**
• **AUBUCHON, David**
**Mission Viejo, California 92692 (US)**
• **VANDEN DRIES, John**
**San Clemente, California 92673 (US)**

(74) Representative: **Hoffmann Eitle**
**Patent- und Rechtsanwälte PartmbB**
**Arabellastraße 30**
**81925 München (DE)**

(56) References cited:
**EP-A1- 3 632 482      US-A1- 2004 054 374
US-B2- 8 535 333      US-B2- 9 039 761**

**Description**

**CROSS-REFERENCE TO RELATED APPLICATIONS**

This application claims the benefit of and/or priority to US provisional application 63/219,480 filed July 8, 2021.

**FIELD**

The present application relates to methods and apparatuses for the delivery of ocular implants, including apparatuses with improved retention of an ocular implant within the apparatuses and methods of accomplishing the same.

**BACKGROUND**

Extended-release drug delivery systems in the form of biodegradable intraocular implants, such as extruded implants, can provide an effective means for delivering therapeutically effective levels of a drug to the eye of patient suffering from an ocular condition. Various sites exist in the eye for implantation of a drug delivery system, including the vitreous, anterior and posterior chambers, as well as the intraretinal, subretinal, intrachoroidal, suprachoroidal, intrascleral, episcleral, subconjunctival, subtenon, intracorneal and epicorneal spaces. The particular site chosen for the drug implant may depend on the ocular condition and the region of the eye affected by the condition, and/or on the drug to be delivered.

Intraocular implants are often administered to a patient through an implant administration device, such as a syringe needle. Implants, once inserted into the implant administration device, have the potential to fall out of the needle due to stresses from handling of the implant administration device with the implant inside. A hard plug may be used to secure an ocular implant within the needle. However, hard plug systems for securing an implant within a needle can damage the implant or weaken the needle.

There is need in the art for safe and reliable retention systems that allow for convenient, controlled, and minimally-invasive intraocular implant delivery.

There is need in the art for safe and reliable retention systems that allow for convenient, controlled, and minimally-invasive intraocular implant delivery.

Further information helpful for understanding the present invention can be found in the following documents:

EP 3 632 482 A1 relates to a system for treating an eye of a patient in need, the system comprising: a delivery device comprising: a housing sized to be held by an operator and comprising an actuator and a distal nose cone; a needle coupled to and extending distal from the nose cone, the needle having a lumen and a beveled distal end defining a distal opening having a sharpened tip and heel proximal the distal opening; and a retention plug positioned within and spanning the lumen of the needle proximal to the heel and an intraocular implant positioned within the lumen of the needle proximal to the retention plug.

US 9 039 761 B2 relates to ocular implant delivery assemblies which include a cannula having a lumen extending therethrough, a proximal end, a proximal end opening, a distal end, a distal end opening, and a lumen extending through the cannula.

US 8 535 333 B2 relates to a delivery device and methods for delivering an ocular implant into an eye.

US 2004/054374 A1 relates to an apparatus and methods for delivering ocular implants or microimplants.

**SUMMARY**

The present invention is defined by the appended independent claims. The dependent claims are directed to optional features and preferred embodiments.

In aspects of the present invention, there are provided a system for treating an eye of a patient in need and a method of retaining an implant in an implant administration device according to the independent claims.

Preferred embodiments are set out in the dependent claims and in the remaining part of the description.

In light of the above, new drug delivery devices, systems, and methods would be beneficial, particularly for delivering therapeutic agents to the eye. It would be particularly advantageous to provide improved retention of the implant within the delivery device until it is ready to be injected in the eye and in a manner that is safer to the eye during the administration.

In an aspect, provided is a system for treating an eye of a patient in need that includes a delivery device having a housing sized to be held by an operator, an actuator and a distal nose cone. The delivery device also includes a needle coupled to and extending distal from the nose cone. The needle has a lumen and a beveled distal end defining a distal opening having a sharpened tip and heel proximal the distal opening. The system further includes a retention plug positioned within and spanning the lumen of the needle proximal to the heel. The retention plug is formed from a flowable retainer solution of hydroxypropyl methylcellulose (HPMC) in water having a concentration that is greater than 2.5% w:w and less than about 4% w:w. The system further includes an intraocular implant positioned within the lumen of the needle proximal to the retention plug.

The delivery device can further include a push rod operatively coupled to the actuator via a linkage to move the push rod through the lumen and push the implant out from the needle. The push rod can have a length relative to a length of the needle sufficient for a distal end of the push rod to advance past the sharpened tip of the needle upon deployment of the implant using the actuator. The push rod can have a shortened length relative to a length of the needle to avoid a distal end of the push rod advancing past the sharpened tip of the needle upon deployment of the implant using the actuator. The shortened length of the push rod can slow an ejection speed of the implant from the lumen upon deployment using the actuator. The retention plug can further slow the ejection speed of the implant. The retention plug can prevent inadvertent release of the implant from the lumen prior to actuation of the device. The retention plug can slow an ejection speed of the implant from the lumen upon deployment using the actuator. The ejection speed can be no greater than about 25 mm/second at an ejection distance of about 20 mm away from the lumen.

The needle can be sized between 22 gauge and 30 gauge. The needle can have a siliconized external surface and the lumen can be substantially non-siliconized. The HPMC can be F4M grade having about 27.0%-30.0% methoxyl substitution and about 4.0% - 7.5% hydroxypropoxyl substitution. The HPMC can be E4M grade having viscosity grade of 4,000 cP and 28.0% - 30.0% methoxl substitution. The retainer solution can have a viscosity between 6,780 cP and 12,780 cP. The needle can be 22 gauge and the retainer solution dispensed within the lumen of the needle as a dispensed mass greater than 300 $\mu$g and less than 1000 $\mu$g. The needle can be 28 gauge and the retainer solution dispensed within the lumen of the needle as a dispensed mass greater than 100 $\mu$g and less than 300 $\mu$g.

The delivery device can further include a cylindrical, distal extension projecting distal from the nose cone and covering at least a proximal end region of the needle, the distal extension defining an exposed working length of the needle. The distal extension can have an outer diameter that is greater than a maximum outer diameter of the exposed length of the needle. The outer diameter of the distal extension can be sized to remain outside the eye and provide a depth stop for the exposed length of the needle sized to be inserted within an eye. The distal extension can have a length that is 8 mm - 15 mm and the exposed working length of the needle can be 5.5 mm - 6.5 mm. The distal extension and the nose cone can be a plastic material and the needle can be a metal material. The plastic material can be acrylonitrile-butadiene styrene and the metal can be stainless steel. The intraocular implant can include a therapeutic and a biodegradable polymer matrix having at least one biodegradable polymer. The therapeutic can be dexamethasone or bimatoprost. The intraocular implant can be OZURDEX® or DURYSTA®.

In an interrelated aspect, provided is a method of retaining an implant in an implant administration device. The method includes formulating a flowable retainer solution of hydroxypropyl methylcellulose (HPMC); dispensing a dispensed mass of the retainer solution into a lumen of a needle to form a retention plug that is contained within and spanning the lumen of the needle proximal to a distal opening from the needle; positioning an intraocular implant within the lumen of the needle proximal to the retention plug; and positioning a shortened push rod proximal to the implant, the push rod operatively coupled to an actuator of the implant administration device. The retention plug prevents inadvertent release of the implant from the lumen prior to actuation of the implant administration device and works in conjunction with the shortened push rod to slow the ejection speed of the implant from the lumen upon deployment in the eye using the actuator. The needle is 22 Gauge or smaller. The needle has a lumen that is substantially non-siliconized and has a siliconized external surface. The ejection speed is no greater than about 25 mm/second at an ejection distance of about 20 mm away from the lumen. The retainer solution has a concentration that is greater than 2.5% w:w and less than about 4% w:w.

In some variations, one or more of the following can optionally be included in any feasible combination in the above compositions, methods, devices, and systems. More details of compositions, methods, devices, and systems are set forth in the accompanying drawings and the description below. Other features and advantages will be apparent from the description and drawings.

## BRIEF DESCRIPTION OF THE DRAWINGS

These and other aspects will now be described in detail with reference to the following drawings.

FIG. 1A shows a top-down view of an implant delivery apparatus;

FIG. 1B shows a top-down view of the implant delivery apparatus of FIG. 1A with a safety cap covering the needle;

FIG. 1C shows a side view of the implant delivery apparatus of FIG. 1B with the safety cap removed from the needle;

FIG. 1D is a partially exploded side view of the nose cone and needle assembly of the implant delivery apparatus of FIG. 1C;

FIG. 1E is a partially exploded side view of the housing, linkage, and actuating lever of the implant delivery apparatus of FIG. 1C;

FIG. 1F is a cross-sectional view of the implant delivery apparatus;

FIG. 2 shows an enlarged perspective view of the linkage of the implant delivery apparatus shown in FIG. 1E;

FIG. 3A shows an enlarged perspective view of the actuating lever of the implant delivery apparatus shown in FIG. 1E;

FIG. 3B shows an enlarged perspective view of the actuating lever of FIG. 3A;

FIGs. 4A-4B show top and side views of a needle tip according to an implementation;

FIGs. 5A-5B show top and side views of a needle tip according to another implementation;

FIG. 6A shows a side view of a nose cone having a distal extension and needle extending beyond the distal extension;

FIG. 6B shows a detail view of a needle assembly incorporating the distal extension;

FIG. 7 shows a cross-sectional, partial view of a safety tab engaged with a linkage;

FIG. 8 shows a cross-sectional, partial view of the needle assembly of FIG. 6B showing a push rod, implant, and a retention plug within the needle bore;

FIG. 9A shows common dispense pressure range for different viscosities of HPMC solutions dispensed by dispense tips having 0.00350" and 0.00400" inner diameters to achieve a dispense mass range of 125 $\mu$g - 275 $\mu$g in the injection needle;

FIG. 9B shows lack of common dispense pressure range for different viscosities of HPMC solutions dispensed by dispense tips having 0.00450" and 0.00400" inner diameters to achieve a dispense mass range of 125 $\mu$g - 275 $\mu$g in the injection needle;

FIG. 10A shows plug push-out force of siliconized and non-siliconized needles having an HPMC implant retention system;

FIG. 10B shows actuation force of siliconized and non-siliconized needles of FIG. 10A with black line showing dip-coated siliconized needles, blue line showing sprayed-coated siliconized needles, and red showing non-siliconized needles;

FIG. 11A shows a typical implant ejection pattern of an applicator having a standard push rod length (Group 1);

FIG. 11B shows a typical implant ejection pattern of an applicator having 1 mm shortened push rod and HPMC implant retention plug within the needle bore (Group 2);

FIG. 11C shows a typical implant ejection pattern of an applicator having 2 mm shortened push rod and HPMC implant retention plug within the needle bore (Group 3);

FIG. 11D shows an example of an implant that did not detach from the needle tip of a Group 3 applicator upon actuation;

FIG. 12A shows an individual value plot for initial speed in mm/second from an applicator having standard push rod length (Group 1) compared to applicators having shortened push rods and HPMC implant retention plugs (Groups 2 and 3);

FIG. 12B shows an individual value plot for final speed in mm/second from an applicator having standard push rod length (Group 1) compared to applicators having shortened push rods and HPMC implant retention plugs (Groups 2 and 3) where implants that stayed above 10 mm/s in the last frame (orange circles), reached a safe speed of 10 mm/s in the last frame (green circles), or did not separate from the needle (red circles);

FIG. 12C shows the average ejection distance in mm from an applicator having standard push rod length (Group 1) compared to applicators having shortened push rods and HPMC implant retention plugs (Groups 2 and 3);

FIGs. 13A-13D show a steps in a technique to visualize implant delivery in the vitreous cavity;

FIGs. 14A-14C show categorization of implant position relative to the eye anatomy;

FIG. 15A is a representative image of an implant positioned in the P1 position in the vitreous chamber in contact with the *pars plana;*

FIG.15B is a representative images of an implant positioned in the P2 position vitreous chamber anterior to or on the equator.

Generally speaking the figures are exemplary and are not to scale in absolute terms or comparatively but are intended to be illustrative. Relative placement of features and elements is modified for the purpose of illustrative clarity.

## DETAILED DESCRIPTION

Described herein are methods and apparatus for delivering solid or semi-solid materials into the eye. Specifically, the methods and apparatus can be used to introduce intraocular implants containing therapeutic or active agents, including bioerodible implants, into various locations within the eye. The apparatus can include an implant retainer that allows for the implant to be delivered in a safe and controlled manner while ensuring the implant is retained within the delivery device until deployment is desired.

More specifically, the device described herein is superior than prior devices for administering biodegradable intraocular implants into the vitreous chamber of the eye due to a retention plug that more securely retains the implant within needle until it is ready to be injected into the eye. The rate of failure is lower than prior implant retention systems such that the delivery device described herein is more reliable and less costly than past delivery devices. The delivery device described herein is also safer to the patient. The retention plug poses no risk in potential ocular contamination of particulate matter like prior retention systems (e.g., systems that rely upon silicone within the needle lumen for retention). The retention plug of the delivery device also dampens implant ejection speeds thereby providing a shorter ejection distance from the device that decreases the risk of retinal injury compared to known systems. The delivery device components work in conjunction with the retention implant to further decrease the implant ejection rate enhancing the safety margin and a concomitantly lower risk of retinal injury. The retention plug is formed from a formulation that has a viscosity range, dispensed mass, and composition calibrated to a particular needle gauge that ensures decreased risk of premature implant release, smooth implant ejection in the absence of excessive force, and a low risk of causing sequela such as glaucoma.

An "intraocular implant" refers to a solid or semi-solid drug delivery system or element that is sized and configured to be placed in an ocular region of the eye, including, for example, the anterior chamber. Other ocular regions of the eye into which an intraocular implant can be placed include the vitreous body, subconjunctival space, and subtenon space. Intraocular implants may be placed in an eye without significantly disrupting vision of the eye. Examples of an intraocular implant include extruded biodegradable filaments, such as a rod-shaped implant produced by a hot-melt extrusion process, comprising a biodegradable polymer matrix and a pharmaceutically active agent, associated with the polymer matrix, and cut to a length suitable for placement in an eye. Intraocular implants are biocompatible with the physiological conditions of an eye and do not cause adverse reactions in the eye. In certain forms of the present disclosure, an intraocular implant may be configured for placement in the anterior chamber, posterior chamber, subconjunctival space, or vitreous body of the eye. Intraocular implants can be biodegradable and may be configured in the form of a cylindrical or non-cylindrical rod produced by an extrusion process. According to some embodiments, the intraocular implant may comprise an active agent effective for treating a medical condition of the eye.

An "intracameral" implant is an intraocular implant that is sized and configured for placement in the anterior chamber of the eye. The anterior chamber refers to the space inside the eye between the iris and the innermost corneal surface (endothelium). An intracameral implant is also an intraocular implant that can fit into the anterior chamber angle (iridocorneal angle) of the eye without contacting the corneal endothelium and thereby without causing corneal trauma, inflammation, or edema, or iris chaffing. One example of an intracameral implant is a hot-melt extruded, biodegradable, rod-shaped filament comprising or consisting of a biodegradable polymer matrix and an active agent associated with the polymer matrix and cut to a length suitable for placement in the anterior chamber of a mammalian eye (for example, a human eye). A rod-shaped intracameral implant can be 0.5 mm to 3 mm in length and 0.05 mm to 0.5 mm in diameter or maximum width in the case of non-cylindrical rods. An intracameral implant is usually between 20 μg and 150 μg in total weight and can fit into the anterior chamber angle (iridocorneal angle) of the eye without contacting the corneal endothelium and thereby without causing corneal trauma, inflammation, or edema, or iris chaffing. For example, the intracameral implant delivered with the present apparatus into the anterior chamber of a mammalian eye, such as a human eye, can be 0.5 mm to 2.5 mm in length, 0.15 mm to 0.3 mm in diameter, and 20 μg to 120 μg in total weight. In some embodiments, the intracameral implant is deliverable through a 27 gauge, 28 gauge, 29 gauge, or 30 gauge needle. The inner diameter of the needle may vary, depending on whether the needle is a standard or ultra (or extra) thin-wall needle. The diameter, width, or cross-sectional area of the implant should be receivable in the lumen of the needle so that the implant can slidably translate through the lumen of the needle.

An "intravitreal" implant is an intraocular implant that is sized and configured for placement in the vitreous body of the eye. The vitreous body of the eye may accommodate implants larger than those used for the anterior chamber. In some embodiments, the intravitreal implant is deliverable through a 21 gauge, 22 gauge, 23 gauge, 24 gauge, 25 gauge, or 26 gauge needle. The inner diameter of the needle may vary, depending on whether the needle is a standard or ultra (or extra) thin-wall needle. The implant is receivable in the lumen of the needle so that it slides through the lumen for deployment in the eye. Intravitreal implants may have diameters of 0.018 inches or less are deliverable through 22 gauge thin-walled needles, and microimplants with diameters of 0.015 inches or less are deliverable through 23 gauge thin-walled needles. Because of the extremely small cross-sectional diameters or areas of these microimplants, the corresponding length may be proportionally larger to provide desired therapeutic dosages of many active agents. Typically, the intravitreal microimplants can be manufactured so as to extend up to about 6 or 7 mm in length or longer. A microimplant of 7 mm or less in length can be useful, at least for placement in the vitreous, as implants of greater length may interfere with a patient's vision.

The terms "ocular condition" and "medical condition of the eye" are synonymous and used interchangeably herein and refer to a disease, ailment, or condition which affects or involves the eye or one of the parts or regions of the eye, including the anterior or posterior regions of the eye. The eye is the sense organ for sight. Broadly speaking the eye includes the eyeball and the tissues and fluids which constitute the eyeball, the periocular muscles (such as the oblique and rectus muscles) and the portion of the optic nerve which is within or adjacent to the eyeball. Non-limiting examples of a medical condition of the eye (i.e., ocular condition) within the scope of the present disclosure include ocular hypertension (or elevated intraocular pressure), glaucoma, dry eye, and age-related macular degeneration. Glaucoma in a patient may be further classified as open-angle glaucoma or angle-closure glaucoma. In one possible method, the patient receiving an intracameral drug-containing implant using an apparatus according to this disclosure may have or be specifically diagnosed with primary open-angle glaucoma. A given patient having open-angle glaucoma may have low, normal, or elevated intraocular pressure. Other forms of glaucoma within the present disclosure include pseudoexfoliative glaucoma, developmental glaucoma, and pigmentary glaucoma. Other examples of a medical condition of the eye within the scope of the present disclosure includes diabetic macular edema (DME), macular edema, noninfectious uveitis, and other disorders affecting the back segment of the eye.

"Associated with a biodegradable polymer matrix" means mixed with, dissolved and/or dispersed within, encapsulated by, surrounded and/or covered by, or coupled to.

The term "biodegradable," as in "biodegradable polymer" or "biodegradable implant," refers to an element, implant, or a polymer or polymers which degrade in vivo, and wherein degradation of the implant, polymer or polymers over time occurs concurrent with or subsequent to release of the therapeutic agent. A biodegradable polymer may be a homopolymer, a copolymer, or a polymer comprising more than two different structural repeating units. The terms biodegradable and bioerodible are equivalent and are used interchangeably herein.

"Active agent," "drug," "therapeutic agent," "therapeutically active agent," and "pharmaceutically active agent" are used interchangeably herein to refer to the chemical compound, molecule, or substance that produces a therapeutic effect in the patient (human or non-human mammal in need of treatment) to which it is administered and that is effective for treating a medical condition of the eye.

The term "patient" can refer to a human or non-human mammal in need of treatment of a medical condition of the eye.

The term "treat", "treating", or "treatment" as used herein, refers to reduction, resolution, or prevention of an ocular condition, ocular injury or damage, or to promote healing of injured or damaged ocular tissue. A treatment is usually effective to reduce at least one sign or symptom of the ocular condition or risk factor associated with an ocular condition.

As used herein, "self-sealing" methods of delivering intraocular implants into the eye refers to methods of introducing implants through a needle and into desired locations of a patient's eye without the need for a suture, or other like closure means, at the needle puncture site. Such "self-sealing" methods do not require that the puncture site (where the needle penetrates the eye) completely seal immediately upon withdrawal of the needle, but rather that any initial leakage is minimum and dissipates in short order such that a surgeon or another equally skilled in the art, in his or her good clinical judgment, would not be compelled to suture or otherwise provide other like closure means to the puncture site.

Unless otherwise indicated herein, viscosity measurements (indicated in cP) are at 25 °C and 100 rpm (see Example 1).

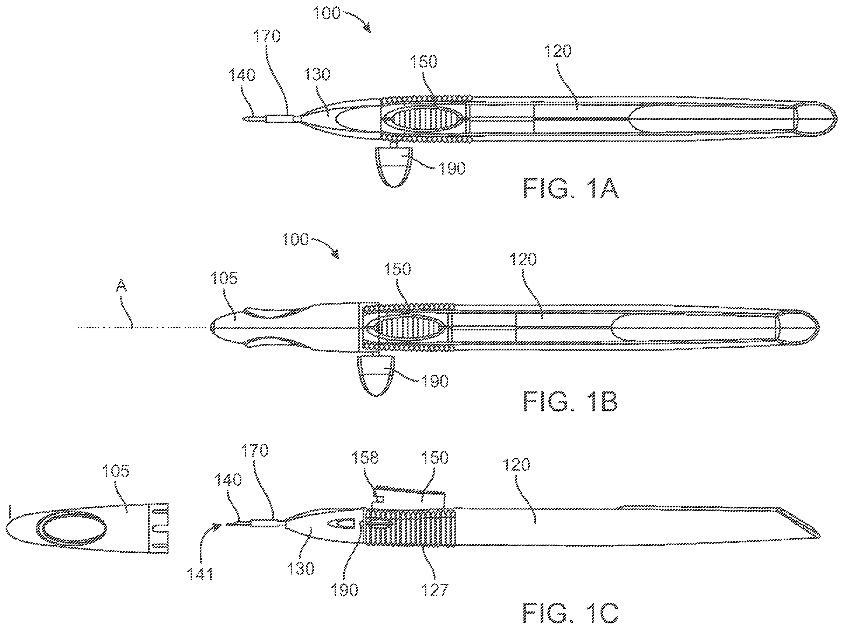

FIGs. 1A-1C illustrate an implementation of an implant delivery apparatus 100 from the top with and without a safety cap 105 (FIG. 1A and 1B, respectively) and from the side with the safety cap 105 removed (FIG. 1C). The delivery apparatus 100 can include a housing 120 with nose cone 130 attached to and extending from a distal end region of the housing 120. A needle 140 having a beveled tip 141 can extend distally from the nose cone 130. The housing 120 can include an actuator such as an ejector button 150 configured to eject an implant loaded within the needle 140 for deployment in a patient's eye at a desired location.

The apparatus 100 can be ergonomically configured for easy gripping and manipulation, and have a general overall shape similar to a conventional pen or other writing instrument. The apparatus will typically be grasped by the user between the thumb and the middle finger and can incorporate one or more features to improve grip and user comfort. For example, the housing 120 can include tactile ridges 127 in selected areas such as around the ejector button 150 where the thumb and middle finger of the user are in contact the apparatus, to provide a more secure grip and feel to the user. Ejector button 150 itself can be provided with tactile grooves 153 on the button surface where the finger (or thumb depending on preference) typically contacts the button, also providing for a more secure grip and feel for the user. The ridges 127 can be rubberized for comfort and to provide non-slip surfaces by which to firmly grip and hold the device.

The housing 120 can be formed of two half sections 121 and 122. In some embodiments, these sections are configured to snap-fit together, although other known methods of attaching the two halves together are contemplated, including, e.g., gluing, welding, fusing, etc. Alternatively, the housing could be singularly molded. Label plate 123 can also be provided, which likewise can be snapped onto or otherwise secured to, the housing.

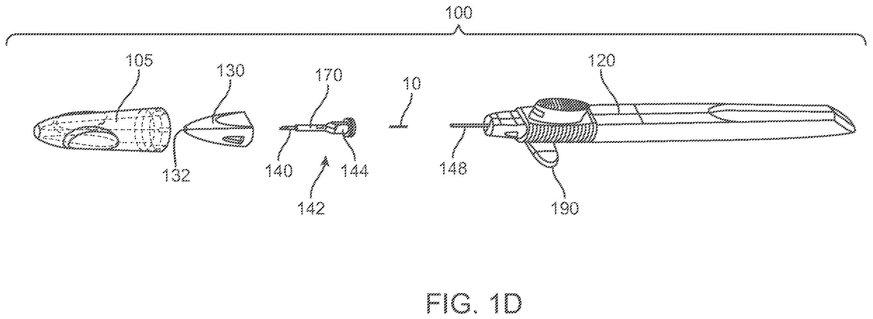

The distal nose cone 130 can be integral with the housing 120 or can be manufactured as a separate piece that is secured to the housing 120. Specifically, nose cone 130 can be secured to collar 124 of housing 120 (see FIG. 1D). Nose cone 130 can receive needle assembly 142 including needle 140 and needle hub 144. The hub 144 and hub base 145 extending proximally from the hub 144 are configured for receipt and securement within nose cone 130, with needle 140 extending through nose cone hole 132. The needle lumen 125 is in communication with inner passageway 143 of the hub 144, such that implant 10 can be passed through the inner passageway 143 of the hub 144 and loaded into the needle lumen 125. Because the implant 10 is pre-positioned within the needle lumen 125 no transfer step is necessary in order to deploy the implant 10 using the apparatus 100. The needle lumen 125 can be sized to receive at least a portion of a plunger 146. The plunger 146 can have a distal push rod 148 extending distal to a linkage 160. The push rod 148 is configured for slidable receipt within the needle lumen 125, and of sufficient length to displace a loaded implant 10 retained with the needle lumen 125 and eject it from the needle tip 141. In some implementations, the push rod 148 can project beyond the distal tip 141 to aid in deploying the implant from the distal tip 141. The distal end of the push rod 148 can project beyond the distal tip 141 by about 0.5 mm to about 1.0 mm. For example, the push rod 148 can be 27.64 mm so that upon actuation of the device and full extension of the push rod 148 through the needle at least 0.5 mm of the push rod 148 extends distal to the distal-most tip of the needle 140. The length of the push rod 148 can vary and need not project beyond the distal tip 141 of the needle to deploy the implant. In some implementations, the push rod 148 is shortened to slow an ejection speed of the implant, which will be described in more detail below. The apparatus 100 having a bevel and elongated opening from the lumen 125 can deploy the implant 10 from within the needle lumen 125 by urging the implant 10 distal to at least the needle heel just proximal of the opening.

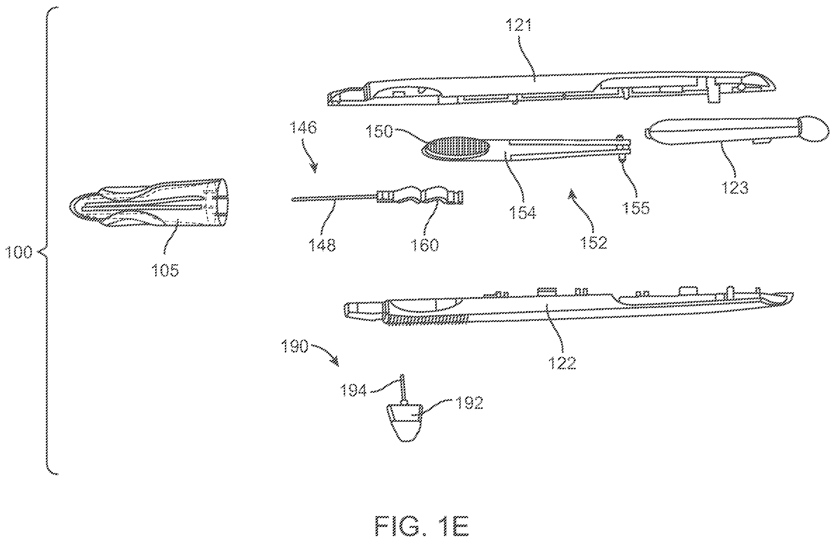

FIGs. 1E-1F illustrate actuating lever 152 and linkage 160, which can be retained within housing 120. Actuating lever 152 can include elongate member 154 having one or more pins 155 extending from the member 154 at one end and ejector button 150 extending from the other end. Pins 155 can extend along an axis A' that is normal to the longitudinal axis A of the needle 140. The pins 155 can be received in corresponding pivot holes (not visible) of the housing sections 121, 122, such that when assembled, the lever 152 can pivot about the pins 155 in a restricted range of motion within the housing 120.

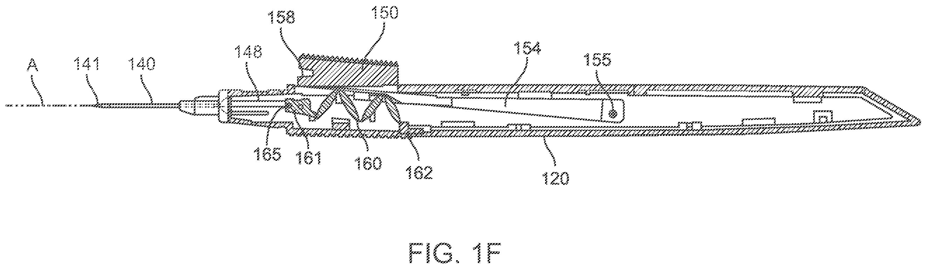

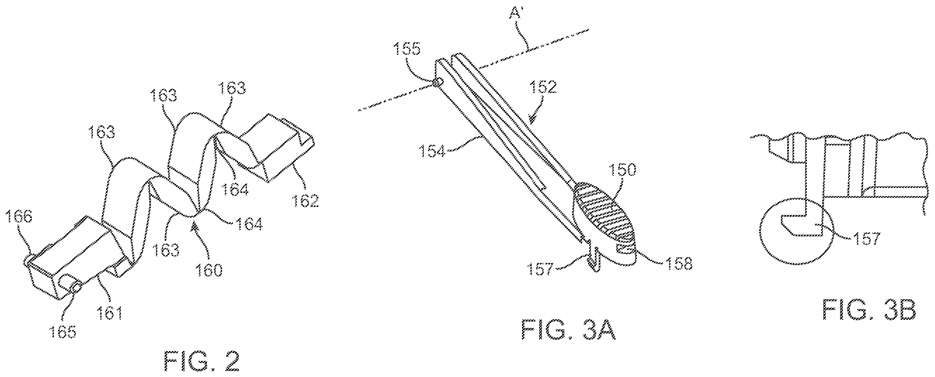

FIG. 1F and also FIG. 2 shows the linkage 160 can include front and rear blocks 161 and 162, with a plurality of joined segments 163 extending therebetween. The segments 163 are sequentially joined to one another. Flexible joints 164 connect the segments 163 to each other and to the front and rear blocks 161, 162. In some embodiments, the linkage 160 is flexible yet resilient, and formed of a contiguous, moldable plastic piece. Portions of the linkage 160 having a relatively thin cross-sectional area of material form flexible joints 164, and disposed between thicker, less flexible segments 163. This allows for flexure of the linkage 160 at the joint locations when a force is applied to the linkage 160. Other known materials are also suitable for the linkage 160, including e.g. shape memory alloys, provided the resultant linkage is capable of lengthwise extension when a force normal to or perpendicular to the length of the linkage is applied. When assembled, rear block 162 is fixedly secured into the housing 120. Guide pins 165, 166 can extend from front block 161 and be received within a guide track of the housing 120. The linkage 160 interacts with the button 150 in a manner that allows a user to depress the button 150 slowly so that advancement of the push rod 148 is gradual with depression of the button 150 to avoid shooting the implant out of the lumen 125 of the needle 140 at a high velocity.

The underside of button 150 of actuating lever 152 can be in contact with the linkage 160 (see FIG. 1F). In operation, depression of button 150 by the user transmits force against the linkage 160 through underside of button 150 in a direction generally normal to the longitudinal axis A of the apparatus. This force is transmitted through the linkage 160, and is converted into a longitudinal force along the longitudinal axis A of the apparatus 100, through flexure of the linkage joints. Because the rear block end 162 of the linkage 160 remains fixed to the housing 120, this action results in translational motion of the free, front block end 161 of the linkage 160 in the direction away from the fixed rear block 162 of the linkage 160. This translational movement of the front block 161 of the linkage 160, in turn, pushes push rod 148 through the lumen 125 of needle 140. Where an implant 10 is loaded and retained within the needle lumen 125, the motion of the push rod 148 in turn ejects the implant 10 from the needle tip 141.

FIGs. 3A-3B show the button 150 can also include a tab 157 extending below a plane of a lower surface of the button 150. The tab 157 can engage with tab slot (not shown) of the housing 120. The engagement between the tab 157 and the tab slot can retain the actuating lever 152 in a locked, depressed condition, after deployment of the implant 10. The tab 157 can include a detent which, when engaged in slot will provide an audible click, signaling the user that the implant 10 has been deployed from the apparatus 100. In other implementations, the actuation of the button 150 provides some tactile feedback to the user that is inaudible or only somewhat audible to the patient so as to avoid causing inadvertent movements by the patient during deployment of the implant. The geometry of the lower surface of the tab 157 can be rounded and the tab 157 can be moveable so that it flexes inward as the tab 157 moves downward through the housing back the tab slot. Once the tab 157 moves a distance past the tab slot, the tab can return to its natural position and engage with the tab slot. The geometry and motion of the tab 157 relative to the tab slot provides an engagement that is dampened so as to provide at least some audible and tactile feedback to a user that the push rod 148 is in a fully deployed configuration relative to the needle 140 without a startling snap.

The needle 140 can be substantially straight along a longitudinal axis A of the apparatus 100 between the nose cone 130 to the distal tip 141. The needle 140 can be a standard surgical needle formed of stainless steel in a variety of gauges. The gauge will be chosen such that the inner diameter of the needle 140 lumen 125, or bore, will correspond to or accommodate the outer diameter of the implant to be delivered, with enough tolerance such that the implant can be received into and subsequently ejected from the lumen 125. The needle 140 can be a standard surgical needle having a luer lock fitting on its hub, which can be received and secured to a corresponding luer fitting provided on the end of the housing 120.

The intraocular implant delivery apparatus according to this disclosure can comprise, for example, a 22 gauge, 23 gauge, 24 gauge, 25 gauge, 26 gauge, 27 gauge, 28 gauge, 29 gauge, or 30 gauge needle. The needle can further be a standard wall, thin-wall or ultra thin-wall needle. Finer, higher gauge needles, such as 28 gauge, 29 gauge, or 30 gauge needles, can be useful for injections into the anterior chamber of the eye to create a small, self-sealing wound and to avoid fluid leakage from the eye. In some embodiments, the distal end of the needle (i.e., the end that extends longitudinally from the distal end of the apparatus housing) is beveled to create a sharp pointed tip that may easily penetrate the tissue of the eye. The intraocular implant delivered by the device should be receivable in and deliverable through the lumen of the needle. In one embodiment, the apparatus comprises a 28 gauge needle. In a more specific embodiment the apparatus comprises a 28 gauge needle with a wall that is 0.0015 inches to 0.0035 inches thick (i.e., about 0.038 mm-0.089 mm thick). In one embodiment the implant delivery apparatus comprises a 28 gauge needle with a wall that is 0.0015 inches to 0.00225 inches thick (i.e., about 0.038 mm-0.057 mm thick). In one embodiment the 28 gauge needle has a wall that is 0.0020 inches to 0.0030 inches thick (i.e., about 0.051 mm-0.076 mm thick). In one embodiment the 28 gauge needle has a wall that is 0.0020 inches to 0.00225 inches thick (i.e., about 0.051 mm-0.057 mm thick). In one embodiment the apparatus comprises a 28 gauge needle with a wall thickness of about 0.0020 inches. In other embodiments, the apparatus comprises a 27 gauge needle with a wall that is 0.0015 inches to 0.0040 inches thick (i.e., about 0.038 mm-0.102 mm thick), or more specifically, that is about 0.0025 inches thick. Another embodiment provides for an apparatus according this disclosure comprising a 29 gauge needle, wherein the needle wall is 0.0015 inches to 0.0030 inches thick (i.e., about 0.038 mm-0.076 mm thick), or more specifically, about 0.0020 inches to about 0.0025 inches thick. A 30 gauge needle may have a wall that is 0.0020 inches to 0.0025 inches thick.

In other embodiments, the apparatus comprises a 22 gauge, 23 gauge, 24 gauge, or 25 gauge needle. As may be appreciated by one of skill in the art, the diameter of the implant may be increased or decreased (e.g. during production) in correspondence with the inner diameter of the needle that is present on the implant delivery device to produce an implant that can be received in and slidably translated through the lumen of the needle.

One example of an intraocular implant suitable to be received in and delivered by the present apparatus is a rod-shaped, biodegradable, drug-containing implant formed by an extrusion process having a diameter and a length that is suitable for delivery through the needle and suitable for placement in the anterior chamber of the eye. Such implants may be referred to as intracameral implants. According to some embodiments, the rod-shaped intracameral implant contained by the apparatus is 0.5 mm to 3 mm in length and 0.05 mm to 0.3 mm in diameter (or maximum width in the case of non-cylindrical rods). In one embodiment, the intracameral implant is 0.5 mm to 2 mm in length and 0.05 mm to 0.25 mm in diameter. For example, the intracameral implant can be 100 $\mu$m to 200 $\mu$m ($\pm$10 $\mu$m) in diameter.

If desired, more than one implant can be placed in the eye during a single administration using an applicator. For example, two implants may be placed in the eye (e.g., anterior chamber, vitreous chamber) to deliver the same or a larger dose of the therapeutic. As an example, an eye may be dosed with 20 µg of Bimatoprost in the anterior chamber by placing two 10-µg implants (each containing 20% bimatoprost by weight) in the eye simultaneously rather than using a single 20-µg implant. Using two smaller implants may possibly improve the tolerability of the implants in the eye. Depending on the overall size of the implant within the lumen of the needle, the arrangement of delivery apparatus components can be adjusted to accommodate the presence of the multiple implants.

In addition to needle dimensions, additional modifications to both the needle tip and in particular methods of insertion can further aid successful self-sealing methods of implantation. A typical problem when inserting a needle into any tissue is the phenomena of "coring" of the tissue, where the insertion actually cuts a cylindrical section of tissue that enters the needle lumen. Such coring when it occurs in the eye can exacerbate leakage of eye fluid through the injection site. By approaching the eye tissue at more of an angle relative to normal, there is a better opportunity for the needle tip penetrate and separate through the tissue layers and reduce coring of the tissue. Additional techniques to further reducing coring and/or excessive leakage are further described herein.

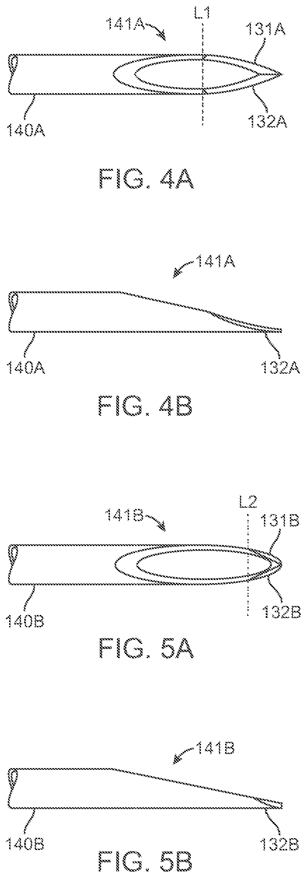

The needle tip 141 itself also can be configured to reduce coring phenomena, for instance, by sharpening certain portions of the bevel tip and dulling others. The needle tip 141 can include side bevels 131a, 132a that extend distally of designated line L1 and constitute approximately one half of the bevel tip and dulled area 133a extending proximally of line L1, constituting the other half of the bevel tip (see FIGs. 4A-4B). The dulled area 133a can be created through conventional polishing techniques known in the art. Where needle tip 141b also includes side bevels 131b and 132b extending distally of designated line L2 and dulled area 133b extending proximally of line L2 (see FIGs. 5A-5B). However, in this embodiment, the side bevels 131b, 132b constitute only about one quarter or less of the bevel tip 141. In each of these embodiments, the sharp side bevels allow for an initial piercing of tissue, but as the tip 141 is further inserted the tissue encounters the dulled areas of the bevel tip 141, which do not have sharp cutting edges, thus promoting separation of tissue layers as the needle is advanced and mitigating against further cutting and possible coring of the tissue.

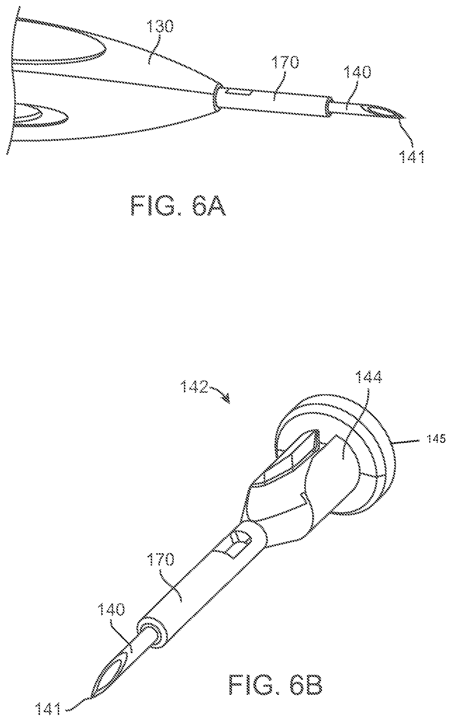

The needle 140 can extend a length distal to the nose cone 130 of the housing 120. The nose cone 130 can incorporate a cylindrical, distal extension 170 that covers at least a portion of the proximal end region of the needle 140 (see FIGs. 6A-6B). The distal extension 170 and nose cone 130 can be integral and/or formed of the same material. In some implementations, the distal extension 170 and the nose cone 130 are integral with one another and formed of Acrylonitrile-Butadiene-Styrene (ABS) plastic. The cylindrical distal extension 170 can have an outer diameter that is smaller than the outer diameter of the nose cone 130 and larger than the outer diameter of the needle 140. The larger outer diameter of the distal extension relative to the needle 140, which can be sized larger than a puncture through which the needle 140 is inserted, allows for the distal extension 170 to act as a visual depth indicator and/or stop that prevents overinsertion of the needle 140 beyond a desired depth. The outer diameter of the distal extension 170 can be uniform between its proximal and distal ends or can vary such that the outer diameter nearest the nose cone 130 is larger than the outer diameter at the distal end. For example, FIG. 6A shows a substantially cylindrical distal extension 170 projecting distal to the nose cone 130 and terminating at a distal opening revealing an exposed length of the needle 140. FIG. 6B shows the substantially cylindrical distal extension 170 projecting distal to the hub 144 and hub base 145. The hub 144 and hub base 145 are coupled inside the nose cone 130 such that only the distal extension 170 extends outside the nose cone 130. The distal extension 170 extending outside the nose cone 130 can have an exposed length that is between 8 mm and 15 mm, such as about 10 mm. The distal extension 170 that extends outside the nose cone 130 can have an outer diameter that is between about 1.0 mm and 2.0 mm, such as about 1.5 mm. The desired depth of penetration of the needle 140 can vary depending on the anatomy. In some implementations, the needle 140 is inserted into the anterior chamber and the desired depth is between about 4 mm to about 7.5 mm measured from the distal-most tip of the needle 140 to the corneal surface where the needle first penetrates the eye. Thus, the distal extension 170 can extend over the proximal end region of the needle 140 such that only between about 4 mm to about 7.5 mm of the needle 140 is exposed distal of the distal extension 170. In other implementations, the needle 140 is inserted into the vitreous and the desired depth is between about 5 mm to about 7 mm measured from the distal-most tip of the needle 140 to the scleral surface where the needle first penetrates the eye. The distal extension 170 can have a length such that the exposed length of the needle 140 between the distal end of the distal extension 170 and the distal-most end of the beveled tip 141 is 5.5 mm to about 6.5 mm, such as about 6.0 mm. The exposed length allows for the needle 140 to be inserted for deployment of the implant 10 without the risk of inserting the needle 140 too far into the eye. In some embodiments, the distal extension 170 is designed so that only the desired depth of penetration into a particular anatomical space is allowed by exposing a length of the needle that corresponds to the desired depth of penetration. The desired penetration depth may vary for different therapies being injected. The distal extension 170 can be atraumatic and free of square edges so that the outer surface of the eye is protected from injury even upon full insertion of the exposed region of the needle 140 within the eye.

The distal extension 170 can provide a depth stop function without coming into contact with the implant positioned inside the bore at any point during use. Some depth stop sleeves are arranged relative to the needle that the silicone of the sleeve comes in contact with the implant positioned inside the bore. As an example, the depth stop can be a silicone sleeve positioned on an outer surface of the needle 140. The sleeve can be fixed in place by a distal edge of the silicone sleeve extending through a small slot in the needle wall into at least a portion of the needle bore. This distal edge protruding into the needle bore through the slot can apply a normal force on the implant providing friction retention of the implant within the bore. The sleeve can provide two functions in that it prevents overinsertion while also retaining the implant in place within the needle bore by passive friction between the implant and the distal edge of the sleeve that contacts the implant. As the implant is urged through the needle bore by the push rod of the applicator, the protruding edge of the outer sleeve can be forced by the implant laterally outward from the slot thereby allowing implant delivery through the needle bore. Such a retention system relies upon lateral motion of the outer sleeve relative to the needle wall to accommodate the implant deployment through the needle bore. The distal extension 170, in contrast, provides only a depth stop function without contacting the implant. The implant can instead be retained within the needle bore by a polymer retention plug fully housed within the needle bore distal to the implant. This will be described in more detail below.

Conventional hypodermic needles can be coated with hydrophobic materials like silicone oil to improve glide through tissues. The outside surface of the needle 140 of the apparatus 100 can be coated such as with a silicone oil coating. This "siliconized" outside surface provides lubricity and a very low coefficient of friction for the surface of the needle that decreases risk of unintended trauma at the point of cannular insertion into the eye. The needle can be siliconized, for example by electro spray-coating with silicone oil so that the external surface of the needle is siliconized and the inside surface (i.e., the lumen) is non-siliconized or siliconized only within the bevel near the opening to the lumen. Spray-coated siliconized needles can include a small amount of silicone migrating from the bevel and entering the lumen, but not so much that the silicone coats a surface of the lumen where the retention plug may be positioned. Dip-coating needles with silicone can coat the outside surface of the needle as well as allow silicone to enter the bore of the needle via capillary forces and coat the inside surfaces of the needle. This coating of the inside surfaces can be particularly problematic with larger needle sizes (e.g., 22G). Siliconizing the needle bore can negatively impact the robustness of the retention plug within the needle and increase the likelihood of plug failure or failure to retain the implant within the bore prior to actuation. Spray-coating needles avoids substantially siliconizing the needle bore and focuses the silicone to the outside surfaces so that the silicone and the retention plug do not come into substantial contact with one another. The retention plug can have improved adherence to the non-siliconized surface of the needle bore compared to if the surface were coated with silicone. The implant retention plug will be described in more detail below.

The silicone can cover the entire exposed working length of the needle near where the proximal end of the needle extends outside the nose cone to the distal tip of the needle. The silicone can cover an exposed length of the needle that is less than the entire exposed outer surface. For example, the silicone can cover the distal end region along a length including the distal-most end of the needle including the beveled opening along an outside surface that is about 1.0 mm - about 5 mm away from the heel of the bevel. The silicone can coat the inner surface of the bevel as well as the outer surface of the bevel and still not come into contact with the retention plug contained inside the bore of the needle. Thus, the inner surface of the bevel may be considered an outside or exterior surface of the needle because it is exposed to tissue as the needle passes through the eye. Complete coverage of the surfaces of the needle that come into contact with eye tissue can improve insertion of the needle through that tissue, for example, the sharpened tip of the bevel, without snagging. It should also be appreciated that the needle can be free of any silicone coatings whether on the inside surfaces or the outside surfaces.

The apparatus 100 can be packaged to include a safety cap 105 extending over the needle 140 and secured to the housing 120 (see FIGs. 1B and 1C). This will provide a measure of safety during handling of the apparatus 100. The button 150 or other depression mechanism of the apparatus 100 can include a notch 158 on a distal end region (see FIG. 1D) that receives the rim of the safety cap 105. In this configuration, the safety cap 105 will then also operate to guard against unintentional depression of the button 150 or other depression mechanism and ejection of the implant 10.

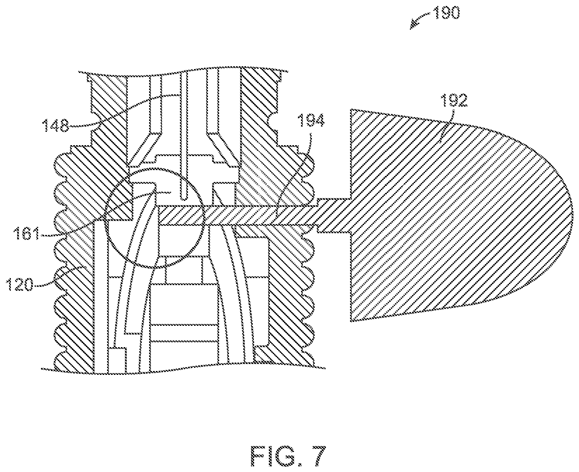

The apparatus 100 can incorporate a safety tab 190 configured to prevent inadvertent actuation of the button 150 (see FIG. 7). The safety tab 190 can include an external gripping portion 192 and an internal post 194. The internal post 194 is configured to prevent actuation of the linkage 160 by the button 150. In an implementation, the post 194 can engage with a region of the front block 161. When the post 194 is engaged with the front block 161, the translational movement of the front block 161 is prevented. Prior to injection, a user can grip the external portion 192 pulling it outward away from the housing 120 disengaging the post 194 from the block 161. The block 161 can then translate upon actuation of button 150. The safety tab 190 can have a discernable color or marking that alerts a user to its presence and that it should be removed prior to actuation of the button 150. For example, the tab 190 can have a first color and the housing 120 can have a second, different color. The tab 190 can also be labeled with words or symbols such as an arrow guiding a user in the direction the tab 190 should be pulled away from the housing 120.

When the apparatus is assembled with the implant, the implant be positioned immediately proximal of the opening at the needle tip. In this fashion, the introduction of air into the eye can be avoided when the implant is ejected, as could otherwise occur were the implant located further within the needle lumen and an air bubble or air pocket allowed to exist between the needle tip and the implant and ejection of the implant were to force the air bubble or air pocket into the eye. The push rod 148 can have a length sufficient to extend through the needle 140 and make contact with the implant 10 to urge the implant 10 from the needle 140. In some implementations, the push rod 148 can have a length sufficient to extend at least partially beyond the distal tip of the needle 140 during actuation so that the implant 10 is expelled entirely from the bore and away from the needle 140. The push rod 148 can extend out the opening within the bevel so that the distal-most end of the push rod 148 projects beyond the distal-most end of the needle 140. This allows the push rod 148 to drive the implant 10 well away from the tip 141 of the needle 140 and into the fluid-filled medium of the eye (e.g., the anterior chamber or vitreous), the implant 10 is less likely to adhere to the end of the needle 140 and thereby the chance that an implant is dragged out of the eye or becomes lodged in the tissues (e.g., cornea or choroid) when the needle is removed. Deposition of the implant in the corneal endothelium, for example, may result in adverse complications. Intracameral implants often separate from the needle tip immediately after ejection. The present device can provide for clean separation of the implant from the device since the push rod 148 may not only drive the implant through the lumen of the needle but may also drive the implant away from the needle tip.

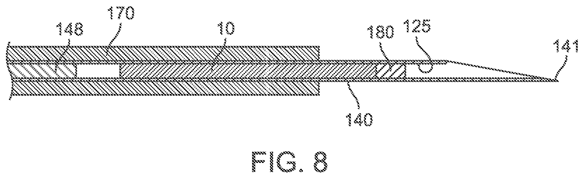

In some embodiments, the apparatus 100 incorporates a retention plug 180 in the needle 140 to prevent inadvertent premature release of the implant 10 from the needle 140 (see FIG. 8). The retention plug 180 can effectively retain the implant 10 within the needle 140, without significantly increasing the ejection force to deploy the implant 10 and also without changing an outer dimension of the needle 140. Minimizing the outer diameter of the needle 140 means the opening into the eye is also minimized. The retention plug 180 can be robust enough to retain the implant 10 within the needle 140 during routine handling of the device, and still allow the implant to be ejected from the device without damaging the implant. The retention plug 180 can be formed so that it is positioned immediately proximal of the opening at the needle tip and the implant positioned immediately proximal to the plug 180. As discussed above, this prevents the presence of an air pocket existing between the distal end of the plug 180 and the tip of the needle.

The length of the push rod 148 in needles incorporating a retention plug 180 can be shortened to accommodate the presence of the plug. The retention plug 180 can be positioned just proximal to the needle heel so the plug 180 is fully inside the bore behind the opening into the needle 140 and the implant 10 can be positioned proximal to the plug 180. The distance the push rod 148 extends through the bore of the needle 140 may be shortened by the length of the plug 180. In some implementations, the implant 10 can be about 7 mm in length and the push rod 148 can be about 28 mm so that the push rod 148 contacts a proximal end of the implant 10 to urge the implant 10 out the opening from the bore. In other implementations, the implant 10 can be about 7 mm in length, the plug 180 can be about 1 mm in length, and the push rod 148 can be about 27 mm allowing additional space for the plug 180. As used herein, a "shortened" push rod is a push rod that has a length less than a standard length push rod in order to accommodate the presence of the plug within the needle bore while also maintaining the relative positions of the implant and the push rod as in the applicator with a standard length push rod. Thus, the shortened push rod is shorter in length by an axial length of the plug spanning the lumen of the needle. The degree by which the push rod is shortened is related to a dispensed mass of the retainer solution into the needle bore and the axial length of the resulting plug within the needle bore. The shortened push rod in an applicator incorporating a retention plug 180 can be shorter than a push rod of a standard applicator without a retention plug 180 by about 1 mm up to less than 2 mm.

The length of the push rod 148 can be varied in order to control the force and speed of the implant ejection thereby minimizing potential tissue damage (e.g., to the retina). In some implementations, the push rod 148 has a length configured to extend out the distal end of the needle 140 upon deployment of the implant so that the distal end of the push rod 148 is positioned past the distal-most tip of the needle 140. In other implementations, the push rod 148 has a length configured to extend near the distal end of the needle 140 upon deployment of the implant, but does not extend past the distal-most tip of the needle 140. The push rod 148 can be between 26 mm and 28 mm. In one embodiment implementation, the push rod 148 is 26.64 mm and configured to eject the implant so that the push rod 148 extends to a distal-most tip of the needle. The push rod 148 in some implementations upon full actuation extends past the distal-most tip of the needle. In other implementations, the push rod 148 upon full actuation stops short of the distal-most tip of the needle and does not extend past the distal-most tip of the needle. For example, the push rod 148 can be a shortened push rod 148 (by less than about 2 mm down to about 1 mm shorter than a standard push rod) to accommodate the presence of a retention plug 180 within the bore. In this implementation, the push rod 148 may stop short of the distal-most tip of the needle upon complete actuation of the push rod 148. Prior to deployment, the push rod 148 can be separated a distance away from the implant 10 positioned in the bore. The distal end of the push rod 148 can be separated from the proximal end of the implant by about 1 mm up to about 2 mm. The shorter push rod 148, the increased distance between the push rod 148 and the implant 10, and the presence of the plug 180 resulted in an improved implant ejection performance compared to applicators incorporating longer push rod 148 and no plug 180. The length of the push rod 148 and/or the presence of the plug 180 can slow the ejection speed of the implant while maintaining a sufficient ejection distance in the eye to provide a reliable and safe deployment of the implant within the vitreous. The push rod can be shortened to accommodate the plug 180 such that the ejection speed of the implant is slowed to avoid striking the retina upon actuation from the device. This is described in more detail below in Example 6.

The average transverse diameter of the vitreous chamber of an adult can be about 25 mm and the average transverse diameter of the anterior chamber can be about 12 mm. In some embodiments, the speed the implant is traveling through the eye once deployed from the delivery device is slow enough that it either does not approach the maximum ejection distance for the anatomical treatment site to avoid a damaging tissue strike or is moving so slowly as to cause no tissue damage if it should reach this maximum distance. The shorter push rod 148 and/or the presence of the retention plug 180 within the lumen can slow or dampen the ejection speed of the implant from the needle to provide a safer injection while still ensuring the implant separates from the needle. An implant that fails to separate from the distal end of the needle can also cause tissue damage, for example, as the needle is withdrawn. The ejection speed of the implants upon deployment from the needle can be no greater than about 35 mm/second upon reaching an ejection distance of about 20 mm away from the distal tip of the needle. In some embodiments, the ejection speed of the implant upon deployment from the needle is even slower upon reaching that ejection distance, including no greater than about 30 mm/second, no greater than about 25 mm/second, no greater than about 20 mm/second, no greater than about 15 mm/second, or no greater than about 10 mm/second. Usually, by the time the implant reaches an ejection speed of about 10 mm/second, the implant will no longer have forward momentum and will instead settle due to the forces of gravity. These ejection speeds and final ejection distances are generally considered suitable to avoid the implant coming into contact with a wall of the eye upon injection, thereby preventing inadvertent tissue injury that could occur, for example, if the implant were to contact the retina if the implant is injected into the vitreous. The ejection speed of the implant can be further dampened for an implant configured to be deployed within the anterior chamber. For example, the ejection speed of the anterior chamber implant upon deployment from the needle can be no greater than about 30 mm/second, no greater than about 25 mm/second, no greater than about 20 mm/second, no greater than about 15 mm/second, or no greater than about 10 mm/second upon reaching an ejection distance of about 10 mm away from the distal tip of the needle. The formulation of the retention plug solution (i.e., concentration, viscosity, etc.), the needle gauge and dispensed mass of the formulation, can be selected to achieve slower ejection speeds and shorter ejection distances upon deployment that in combination with the push rod length, provides for a safer and more reliable deployment of the implant in the eye.

The retention plug 180 can be formed of a single polymer or a mixture of two or more polymers. The retention plug 180 can be formed from a retainer solution containing a water-soluble polymer. The water-soluble polymer can be a cellulose ether, such as hydroxypropyl methyl cellulose (HPMC). HPMC is commercially available, for example, from DuPont Pharma under the brand name METHOCEL®, and WALOCEL™, as well as from Ashland under the brand name BENECEL™. HPMC is also available in different grades having different viscosities based on the nominal methoxy and hydroxypropoxyl substitutions. The HPMC can be F4M grade HPMC having viscosity grade of 4,000 mPa and 27.0% - 30.0% methyoxyl substitution (e.g., 29.0%) and 4.0% - 7.5% hydroxypropoxyl substitution (e.g. 6.0%). The F4M grade HPMC can have a viscosity at 2% w:w in water at about 20 °C that is between 2,663 - 4,970 cP. The HPMC can be E4M grade having viscosity grade of 4,000 cP and 28.0% - 30.0% methoxl substitution. The E4M grade HPMC can have an apparent viscosity at 2% w:w in water at about 20 °C that is between 2,663 - 4,970 cP. Other suitable grades of HPMC include A4M, E4M, F4M, K4M, and J4M, including any of the other grades within the A, E, F, K, and J series. The polymer of the retention plug can also be formed from any of a variety of water-soluble polymers including HPMC as described above, hydroxypropyl cellulose (HPC), polyvidone or povidone, hypromellose acetate succinate (HPMCAS), copovidone, crospovidone, methyl cellulose (MC), methylhydroxyethylcellulose (MHEC), sodium carboxymethyl cellulose (CMC), ethylcellulose (EC), hydroxyethylcellulose (HEC), hydroxypropyl-γ-cyclodextrin, hydroxypropyl-β-cyclodextrin, native cyclodextrin, N-methyl-2-pyrrolidone (NMP), hyaluronic acid, polyethylene oxide, polypropylene oxide, chitosan, agarose, polypeptide, ficoll, hydroxy ethyl methyl cellulose (HEMC), Poly (ethylene glycol) (PEG), N-(2-Hydroxypropyl) methacrylamide (HPMA), Polyoxazoline, Polyphosphates, Polyphosphazenes, Xanthan Gum, Pectins, Dextran, Albumin or natural and synthetic protein. In some embodiments, the retainer solution containing the polymer for forming the retention plug 180 can form viscous solutions having an apparent viscosity between about 6,000 cP and about 13,000 cP, having reasonable adhesion to non-siliconized metals, be water soluble, and biocompatible for use in the eye without causing adverse events. The specification sheets, data sheets, and testing data of these polymers are expressly referred to herein.

The retention plug can be formulated into a polymer retainer solution or polymer retainer gel before it is applied to a needle. The polymer retainer solution or polymer retainer gel can contain the polymer as well as additional excipients for suitable purposes. In an embodiment, certain excipients may be added to the polymer retainer gel or the polymer retainer solution to modify the viscosity of the solution or gel, so that they can be easily applied to the implant administration device. According to an embodiment, an excipient comprising, consisting essentially of, or consisting of isopropyl alcohol and/or water and/or a buffer can be added to the polymer to form the polymer retainer solution or the polymer retainer gel. When used, one or more excipient can be present in the solution or gel in an amount in the range of 0.2% to 10% by weight of the solution or gel, based on the total weight of the solution or gel.

In some embodiments, the polymer retainer solution is a solution of HPMC (e.g., E4M or F4M) that has a concentration greater than 2.5% w:w and less than about 4% w:w. In some implementations, the polymer retainer solution is an HPMC solution in water that is 2.58% F4M-HPMC solution having an apparent viscosity of 6,780 centipoise (cP), 2.8% w:w F4M-HPMC solution having an apparent viscosity of 7,880 cP, 3% w:w F4M-HPMC solution having an apparent viscosity of 10,680 cP, 3.15% w:w F4M-HPMC solution having an apparent viscosity of 12,780 cP. The HPMC solution can have an apparent viscosity greater than or equal to about 6,240 cP and less than or equal to about 12,870 cP, greater than or equal to about 6,780 cP and less than or equal to about 12,760 cP, greater than or equal to about 8,640 cP and less than or equal to about 10,080 cP prior to application to the needle. In some implementations, the viscosity of the retainer solution is between about 7,000 cP and about 13,000 cP. In some implementations, the retainer solution is 3% w:w F4M-HPMC solution having an apparent viscosity of 8,640 cP - 12,760 cP dispensed into a 28 gauge needle as a dispensed mass that is between 125 μg - 200 μg. In some implementations, the retainer solution is 3% w:w F4M-HPMC solution having an apparent viscosity of 8,640 cP - 12,760 cP dispensed into a 22 gauge cannula as a dispensed mass that is between 450 μg - 850 μg.

The HPMC solution can be applied to a needle having different gauges including 22 gauge, 25 gauge, 27 gauge, 28 gauge, or 30 gauge. Generally, the larger the needle, the more HPMC mass that is dispensed to form the plug that is contained within and spans the bore on account of the larger luminal volume. Needles that are 28G may receive a dispensed mass of 100 - 300 μg, such as about 125 - 275 μg, whereas needles that are larger (e.g., 22G) may receive a larger dispensed mass of 300 - 1000 μg, such as about 450 - 850 μg. It should be appreciated that other water-soluble, biocompatible polymers are considered herein for the polymer retainer solution for forming the retention plug. The concentration of polymer retainer solution can be selected to have an apparent viscosity in the ranges described herein and dispensed within the needle bore at a dispensed mass range described herein as being suitable for the particular needle gauge.

The polymer retainer can be applied to the needle as a solution, gel, or hot melt either by dipping the needle tip into solution or by direct application of the polymer retainer to the tip of needle. In embodiments where the polymer retainer is applied to the needle tip as a solution or gel, the solution or gel solvent can be removed before the needle containing the polymer retainer is inserted into a patient. In embodiments where the polymer retainer is applied as a hot melt, the melt can be cooled after application to the needle, but before the needle containing the polymer retainer is inserted into a patient.

**In** some embodiments, the needle is not coated with the polymer retainer solution by dipping, but rather an amount of the solution is dispensed within the needle bore by a dispensing tip of a controlled dispensing system to achieve a dispensed mass. The controlled dispensing system can be an EFD Ultra 2400 Fluid Dispenser. The dispense tip ID range can be between about 0.00350" and 0.0050". Generally, the dispensing tip has a gauge that is smaller than the gauge of the needle being plugged. For example, the needle being plugged can be a 28 gauge needle and the dispensing tip can be 32 gauge. The needle being plugged can be a 22 gauge needle and the dispensing tip can be 27 gauge.

A dispensed mass of the retainer solution can be deposited directly into the distal end of the needle using the dispense needle and according to parameters such as polymer concentration, retainer solution viscosity, dispense needle gauge, applicator needle gauge, dispense time, dispense angle, dispense pressure, etc. to achieve a desired dispensed mass suitable for achieving a retention plug that fully plugs the bore of the needle. The dispensed mass of the retainer solution can vary depending upon the concentration and apparent viscosity of the retainer solution as well as the needle gauge being plugged. In some implementations, the dispensed mass of the retainer solution having an apparent viscosity of between 8,640 cP - 12,760 cP to plug a 28 gauge needle can be greater than 100 μg and less than 300 μg, such as between about 125 - 275 μg. The dispensed mass of the retainer solution having an apparent viscosity of about 10,080 cP to plug a 22 gauge needle can be greater than 300 μg and less than 1,000 μg, such as a range of about 650±200 μg.

The dispense parameters can vary depending on the needle gauge being plugged and the solution being dispensed. The dispense pressure used to deliver the mass can be between 30 psi and 75 psi, such as between about 45 and 55 psi. The dispense time can be between 1.0 and 3.0 seconds, such as between about 1.4 and 2.8 seconds. The dispense angle can be between 5 and 45 degrees, such as about 30 degrees. In an implementation, for a viscosity range of retainer solution that is between 6,780 cP and 7,880 cP and a dispensing tip ID range that is 90 - 100 μm, the pressure range can be between 47 - 51 psi to achieve a dispensed mass of between 125 - 275 μg.

The solution following dispensing can be left to dry (e.g., at room temperature) to form a plug within the bore. Generally, the plug is formed proximal to the heel of the needle and fully inside the bore. The plugging parameters are selected to ensure complete plugs are formed that fully span the bore of the needle as opposed to simply coating the walls. In some embodiments, the needle is siliconized with sprayed-on silicone oil so that the silicone coats the outside surface of the needle and avoids siliconizing the lumen of the needle where the retention plug is to adhere or attach. The retainer plug attaches better to a non-siliconized surface. However, the retention plug can attach too well and negatively impact the functionality of the applicator (e.g., increasing actuation force to deploy the implant). Thus, the siliconization process allows for small amounts of silicone oil to migrate down the bevel of the needle by capillary force. In some embodiments, to mitigate the ingress of the silicone oil into the lumen, the bevel is oriented away from the spray nozzle. This technique results in a fully siliconized outside surface of the needle and just a small amount of silicone oil that settles on the bevel and in the opening of the lumen. The amount of silicone oil that migrates down the bevel in needles spray-coated in this manner is less than the amount of silicone oil that migrates in dip-coated needles thereby providing better adherence of the polymer retainer plug to the needle wall than dip-coated needles, but does not adhere so strongly that the plug impacts the usability of the device like the fully non-siliconized needles. The silicone coating can cover the entire exposed length of the needle (about 6 mm along an outer surface of the needle) or length that is less than the exposed length of the needle as described elsewhere herein.

A retention plug that retains an implant inside a syringe needle can vary in its effect on actuation force. Relevant parameters that can impact the suitability of a retention plug as a retention system for a syringe needle applicator and its impact on actuation force from the syringe needle can vary including molecular weight of the polymer, concentration and viscosity of the retainer solution as well as the dispensed mass of the deposited retainer solution, which can vary depending on the inner dimension, dispense pressure, and dispense time of the controlled dispensing system used. The dispensed mass can be impacted by the retainer concentration and/or viscosity. For example, a higher concentration retainer solution may have a viscosity that the dispense pressure, dispense time, and/or inner dimension of the dispensing needle may need to be modified to achieve the same dispensed mass of a lower concentration retainer solution within the same size needle. Some retainer solutions are too viscous for a particular needle size and can form a plug that is too hard and increases actuation force needed to deploy the implant that the device is not suitably usable. Other retainer solutions are not viscous enough for the needle size used and form a plug that only coats the inner walls of the needle leaving the aperture at least partially open and/or fails to prevent the implant from falling out during transportation and handling. The viscosity of the retainer solution can be adjusted to provide different functional characteristics to the resulting plug that may be selected depending on the size of the needle to be plugged. The viscosity of the retainer solution can be lower for a smaller gauge needle and still sufficient to plug the opening, but the same viscosity retainer solution may not be sufficient to plug the opening of a larger gauge needle and instead merely coat the inner walls of the needle or fail to retain the implant. And still further, a retainer solution may have a viscosity sufficient to fully close the opening of the needle, but be so viscous that an overly rigid plug forms such that the actuation force needed to push the implant through the plug is too high. Such high viscosity plug formulations can increase the ejection force and thus, the speed of the implant as it shoots out the needle, significantly increasing the risk of retinal injury.

Viscosity of the retainer solution can be adjusted by changing the concentration of the retainer solution and/or the molecular weight of the polymer. For example, an HPMC starting material having a higher molecular weight may be selected to create an HPMC solution that is more viscous and suitable for a larger size needle, but that may be too viscous for a smaller size needle. The viscosity of the retainer solution can be lowered by selecting a lower molecular weight HPMC such that the same concentration retainer solution is suitable for the smaller size needle, but that may not be suitable for the larger needle size. Viscosity that is too low prevents the plug from effectively retaining the implant within the needle, for example, during transportation and handling. Such low viscosity plugs may also fail to sufficiently slow the implant as it is ejected from the needle and enters the eye. An implant that shoots out of the needle too quickly can travel too far into the eye causing retinal damage. Thus, the plug can be formed from a retainer solution that has a viscosity suitable for a particular needle size in order to retain the implant during handling and also slows down the implant under the same ejection force as it is ejected from the needle to avoid damaging the implant and the eye. A suitable amount of polymer retainer may be used to effectively plug the needle to contain the implant, but still beneficially minimize the amount of actuation force necessary to expel the implant from the needle. The polymer retainer plugs described herein can provide suitable retention and adherence with the needle bore, but do not significantly increase the actuation force required to deploy the implant from the needle. The actuation force on the button to deploy the implant from the needle can be in the range of about 0.5 pound force to 2.0 pound force, or in a range of about 1.0 pound force to about 1.5 pound force, which is similar to the actuation force on the button to deploy the implant when there is no plug present in the needle bore.

The need for frequent intraocular injections in patients means the injections should be easy and convenient to administer within a doctor's office setting. This goal is sometimes at odds with the need to make the injections safe and painless. The plug within the needle bore makes the applicator more user-friendly so that the applicator may be casually handled without fear of the implant slipping out of the needle bore prior to implantation. However, the plug within the needle bore can be too effective at preventing the implant for slipping out of the bore. The retainer solution of the plugs described herein is calibrated to the needle bore size so that the plug that forms fully spans the bore rather than simply coating the walls to prevent the implant from inadvertently falling out of the needle while still being sufficiently frangible that the implant can pass through the bore at the time of actuation and deployment within the eye.

## EXAMPLES

EXAMPLE 1 - EFFECT OF HPMC CONCENTRATION ON PLUG FORMATION AND VISCOSITY

Whether or not a plug forms in a needle bore can be a function of the concentration of the HPMC solution. Hydroxypropyl methylcellulose (HPMC-F4M powder) was mixed with water for injection (WFI) to create different concentrations of liquid HPMC solution, including 1% w:w, 3% w:w, 4% w:w, 5% w:w, and 6% w:w solutions. The 1% w:w solution failed to fully close off the opening in the needle and was discarded from the study. The 6% w:w solution had a viscosity that made it impossible to be dispensed within the time, pressure, and dispensing needle gauge parameters being considered for the process. Table 1 shows the data for certain plugging parameters. The 3% w:w and 4% w:w HPMC solutions were dispensed using a 30 gauge dispensing tip at a pressure of between 50-55 psi and over a time between 1.4-2.8 seconds into a needle that was 22 gauge. The 5% w:w HPMC solution was dispensed using a 27 gauge dispensing tip at a pressure of 75 psi over 2.5 seconds into a 22 gauge needle. Plugging parameters *i and ia* for the 5% w:w HPMC solution were identical except the dispense tip was manually inserted deeper into the 22G needle in parameter *ia* compared to parameter *i*. After visual inspection all plugs at each plugging parameter formed a meniscus and all the implants at each plugging parameter were able to be ejected from the needles. The actuation force experiments show how much force is required to press the actuator button to achieve deployment of an implant. The actuation force of an applicator was assessed as follows. An applicator was arranged in the horizontal position with the button pointing upwards by a fixture under force measuring equipment probe. The fixture keeps the applicator from deforming during the test so that actuation force on the button is measured accurately. The equipment probe is connected to a force gauge and pushes down onto the button of the applicator. The force in pounds (lbf) required to cause the button to travel downward into the housing of the applicator is recorded by the equipment. The actuation force of the various plugs formed according to the different parameters was not significantly different. Each assembly at each plugging parameter also successfully held the implant.

**Table 1**

| Plugging parameter | HPMC Concentration (% w:w) | Dispense Needle (G) | Dispense Time (s) | Dispense Pressure (psi) | Actuation Force (lbf) |
|---|---|---|---|---|---|
| c | 3 | 30 | 1.4 | 50-55 | 1.97 $\pm$ 0.553 |
| d | 3 | 30 | 2.8 | 50-55 | 1.651 $\pm$ 0.193 |
| f | 4 | 30 | 2.6 | 50-55 | 1.610 $\pm$ 0.164 |
| i | 5 | 27 | 2.5 | 75 | 1.675 $\pm$ 0.128 |
| ia | 5 | 27 | 2.5 | 75 | 1.563 $\pm$ 0.130 |

Apparent viscosity of HPMC solutions was measured to assess the effect of different concentrations of HPMC-F4M on the viscosity of the solution. HPMC-F4M powder was mixed with water for injection (WFI) to create different concentrations of liquid HPMC solution and viscosity of the solution determined by rotational viscometry using a Brookfield RVDV-II+ Pro Extra viscometer and a Brookfield spindle #7-RV with a SC4-13RP sample chamber. The measurements were taken at 100 rpm at a controlled temperature of $25.0 \pm 0.1°$C. The tightly capped samples and standards (15 mL) were placed in a water bath or oven/incubator and stabilized at temperature for at least 3 hours for equilibration prior to testing. Table 2 below shows the apparent viscosity of each of the HPMC retainer solutions.

**Table 2**

| HPMC -F4M (% w:w) | Viscosity (cP) |
|---|---|
| 2.58 | 6,780 |
| 2.80 | 7,880 |
| 3.00 | 10,680 |
| 3.15 | 12,780 |

EXAMPLE 2 - TRANSIT TESTING OF VISCOSITY RANGE

The HPMC plugs were tested for their ability to successfully maintain the implants inside the needle bore under transit challenges. Higher viscosity (12,760 cP or 12,780 cP) and lower viscosity (6,240 cP or 6,780 cP) solutions of 3% w:w hydroxypropyl methylcellulose (HPMC-F4M) were formed for plugging 28G needles of Bimatoprost Sustained Release (BIM SR) applicators. The different viscosities were achieved by creating small deviations from the target concentration of 3% w:w HPMC solution until the target viscosity was achieved. As part of the needle plugging process, apparent viscosity of the HPMC solution was measured to ensure the viscosity of the solution was within the verified range for forming a suitable plug.

The plug was formed by dispensing the HPMC solution into the 28 gauge needle (TSK needles, 44642LH) bore using a 32 gauge dispense tip of an EFD Ultra 2400 Fluid Dispenser. The dispense tip was inserted into the needle bore and a controlled mass of the solution dispensed into the needle under a 55 psi dispenser pressure, over a 1.4 second dispense time, and at a 30 degree dispense angle. The solution was left to dry at room temperature to form a plug near the distal opening from the needle. The plugs formed proximal to the needle heel and were fully inside the needle bore. All the plugged needles were visually inspected for the presence of a complete plug that spanned the entire bore. Needles with no plugs or incomplete plugs not spanning the full bore were discarded and replaced. Sixty units were manufactured using HPMC solutions at low specification limit (30 units at 6,240 cP and 30 units at 6,780 cP) and 60 units were manufactured using HPMC solutions at high specification limit (30 units at 12,760 cP and 30 units at 12,780 cP).

The plugged needles were assembled with implants (10 $\mu$g dose bimatoprost) behind the HPMC plugs and retained in place with the applicator plunger. Applicators were packaged in a foil pouch with desiccant and double heat-sealed. Samples were sterilized by gamma irradiation (22.5 - 27.5 kGy). Individual pouched applicators were placed in an SBS carton. The applicators were challenged after being packaged and sterilized by transit testing to verify the functionality (robustness) of the HPMC plug and its ability to retain the implant in the needle. A total of 20 cartons (10 from low viscosity plugged needles and 10 from high viscosity plugged needles) were challenged through transit testing. Ten samples from each type of plugged needle were placed in alternating fashion in corrugated shippers (RSC 200B; 8.9" x 8.1" x 7.3"). Set 1 (n=3) included HPMC solution viscosities at 6,240 cP and 12,760 cP. Set 2 (n=3) included HPMC solution viscosities at 6,780 cP and 12,780 cP. The transit testing was conducted per ASTM D4169-14 including loose load vibration, low pressure test, vehicle vibration, and concentrated impact study.

After transit testing, no damage was observed to the shipping containers and all 120 applicators remained intact in their pouches. The presence of HPMC plug in the needle were examined before and after the transit study. Actuation force results from all 120 applicators were within the specification limit of NMT 5.0 pound force (see Table 3). Implants were present in all testing applicators and the actuation force results from 120 applicators passed specification of NMT 5.0 pound force. The actuation force results for needles plugged using the low and high viscosities were essentially the same and were in the range of 0.9 pound force to 1.5 pound force. Upon actuation, an implant was ejected from all test applicators. All 120 applicators were found to have safety caps and safety tabs securely attached to the applicators. Only 1 unit of 60 units tested from the plugged needles (1 out of 30 plugged needles with HPMC solutions at 6,240 cP) made from HPMC solution viscosity of 6,240 cP was observed with a loose HPMC plug. The high viscosity HPMC solutions did not result in any loose plugs.

**Table 3**

| Specification Limit | Apparent Viscosity of 3% w:w HPMC Solution (cP) | # of Units | Package Integrity | HPMC Physical Appearance | Actuation Force below 5.0 lbf |
|---|---|---|---|---|---|
| Low Specification Level | 6,240 | 30 | No damage | 1 plug outside on the needle tip | Passed |
| | 6,780 | 30 | No damage | All plugs were in place | Passed |
| High Specification Level | 12,760 | 30 | No damage | All plugs were in place | Passed |
| | 12,780 | 30 | No damage | All plugs were in place | Passed |

The transit testing identified 3% w:w HPMC solutions with viscosities of 12,760 cP or 12,780 cP are useful upper limits for viscosity in the manufacture of plugged 28 gauge needles for the apparatus.

EXAMPLE 3 - CHARACTERIZATION OF HPMC DISPENSE MASS

As discussed above in Example 2, the HPMC solutions was dispensed into the needle bore using a dispense tip. The inner diameter (ID) of the dispense tips used to plug the needles were measured on an inspection microscope. An average dispense tip diameter was calculated to be about 115 microns or 0.0045". Factors that affect the amount of the solution dispensed into the needle is according to Poiseuille's equation:

$$Q = \frac{\Delta P \, \pi \, r^4}{8 \, \eta \, l}$$

where $Q$ = Flow Rate, $P$ = Dispense Pressure, $r$ = Dispense Tip Radius, $\eta$ = HPMC viscosity, $l$ = Dispense Tip Length.

The amount of dispensed HPMC is affected by flow rate and dispense time, which is the amount of time that the dispense pressure is applied to the needle. Dispense time and flow rate control the amount of HPMC solution that leaves the dispense tip and enters the needle lumen to form the plug.

Poiseuille's equation was used to calculate the theoretical HPMC dispense mass based on a medium viscosity HPMC solution (10,680 cP) and the calculated average dispense tip ID of 0.0045" (115 $\mu$m). The experimental range of the dispensed mass of HPMC solution was approximately 150 $\mu$g - 300 $\mu$g. Conservative values were substituted for the dispense tip ID variation (100 $\mu$m - 120 $\mu$m) into Poiseuille's equation such that a likely range of the masses was approximately 80 - 325 $\mu$g.

A dispense mass range of 50 - 450 $\mu$g for the 3% w:w HPMC solution having 10,680 cP was selected to be characterized with respect to plug forming capacity in a 28 g ultra-thin wall (UTW) needle. A total of nine different masses were tested for visual appearance of the formed plug and plug push-out force (POF). POF is measured using a texture analyzer and a steel gauge pin. A needle having an HPMC plug spanning the needle bore is loaded with an implant proximal to the plug and fixed to the base of a mechanical tester. The actuator arm is attached to a push rod that is concentrically aligned with the needle bore orifice fixed below. The test can be a compression test in which the actuator arm is lowered and the push rod passes through the needle lumen until it encounters resistance from the implant that is retained by the plug. The actuator is connected to a load cell that measures the force/resistance encountered (i.e., plug push-out force) while the push rod pushes the implant through the HPMC plug. Acceptance criteria included presence of plug in greater than or equal to 90% of the cases, POF greater than or equal to 1 gF assuming *1000g* acceleration, mass of implant being 10 $\mu$g, and about 0.05 gF (F=mass x acceleration; 1000 * 9.81) m/s$^2$ *50e-9 kg = 0.0005 N (0.05 gF)), solution "blob" visible during dispensing into needle, and dispense tip not covered by the excess solution to avoid "stringing". Orifice coverage of 100% by visual inspection under magnification was required for acceptance. Partial plugs were considered failures.

Tables 4 and 5 below show results of the visual inspection and POF testing with a 28 gauge needle. Table 6 shows results of POF testing with a 22 gauge needle. The HPMC dispense masses less than 100 $\mu$g and greater than 350 $\mu$g did not form reliably within a 28 gauge needle per the acceptance criteria. Needle plugging with dispense masses above 300 $\mu$g may not form reliably in a 28 gauge needle due to "stringing," which occurs when the HPMC solution overflows the needle lumen resulting in irregular formation and pulling of the solution out of the needle ID as the dispense tip retracts. When plugs do form, the POF meets the acceptance criteria for 100 - 450 $\mu$g. However, at 300 $\mu$g of HPMC dispense mass in the 28 gauge needle, one sample did approach the measurement system detection limit of 0.5 gF. A higher incidence of "stringing" was observed during subsequent manufacturing campaigns for needles plugged with greater than 300 $\mu$g HPMC solution.

**Table 4**

| Dispensed Mass ($\mu$g) | Sample size | Pass | Fail | Success Rate |
|---|---|---|---|---|
| 50 | 29 | 2 | 27 | 7% |
| 100 | 29 | 28 | 1 | 97% |
| 150 | 29 | 28 | 1 | 97% |
| 200 | 29 | 28 | 1 | 97% |
| 250 | 29 | 27 | 2 | 93% |
| 300 | 29 | 26 | 3 | 90% |
| 350 | 29 | 27 | 2 | 93% |
| 400 | 29 | 17 | 12 | 59% |
| 450 | 29 | 25 | 4 | 86% |

**Table 5**

| Dispensed Mass ($\mu$g) | Sample size | POF (gF) |
|---|---|---|
| 50 | 10 | n/a |
| 100 | 10 | 1.84 $\pm$ 0.51 |
| 150 | 10 | 6.92 $\pm$ 4.03 |
| 200 | 10 | 6.14 $\pm$ 4.35 |
| 250 | 10 | 6.65 $\pm$ 5.56 |
| 300 | 10 | 4.73 $\pm$ 4.97 |
| 350 | 10 | 6.03 $\pm$ 5.72 |
| 400 | 10 | 7.29 $\pm$ 6.97 |
| 450 | 10 | 1.84 $\pm$ 0.51 |

Conservatively, 300 $\mu$g was classified as the upper limit for the ideal mass for a 28 gauge needle and the functional limit for the ideal dispense mass of HPMC solution was tightened at each extreme for a desired specification of 125 - 275 $\mu$g verified by the plug push-out force (POF) testing. The lower limit for the dispensed HPMC mass was determined by the ability of the present solution to form a plug during drying process and the plug adherence strength quantified by POF. The upper limit was determined by the ability of the dispense tip to remain clean for retraction without "stringing".

Dispense pressures that yield HPMC dispense masses within this range are disclosed based on HPMC viscosity and Dispense Tip ID ranges.

The recommended specification limit for apparent viscosity was 8,640 - 12,760 cP. The upper specification limit for viscosity was supported from the transit studies performed. The lower viscosity limit was set at 8,640 cP because during transit testing one of the lower viscosity tests (6,240 cP) lost a plug. Actuation force for low and high viscosities to eject the implant were essentially the same (e.g., 0.9 lbf to 1.5 lbf).

The effect of dispensed mass on plug push-out force was also assessed for larger needle sizes. A 4% w:w HPMC solution having a viscosity of 10,080 cP was dispensed into 22 gauge needles using 0.00803" ID dispenser tip. Table 6 below shows POF in gF for various dispensed mass. The data suggest ideal dispensed mass for 22 gauge needles was 300-1000 $\mu$g. In an implementation, the dispensed mass for 22 gauge needles is a narrower range of 450-850 $\mu$g.

**Table 6**

| Dispensed mass ($\mu$g) | Sample size | POF (gF) | Actuation Force (gF) |
|---|---|---|---|
| 200 | 4 | 11.91 $\pm$ 1.13 | 798.16 $\pm$ 198.78 |
| 400 | 4 | 16.32 $\pm$ 9.15 | 657.99 $\pm$ 96.98 |
| 600 | 4 | 16.24 $\pm$ 3.82 | 781.30 $\pm$ 166.56 |
| 800 | 4 | 11.54 $\pm$ 5.69 | 637.57 $\pm$ 228.39 |
| 1000 | 4 | 23.44 $\pm$ 7.35 | 877.40 $\pm$ 214.91 |
| 1200 | 4 | 25.85 $\pm$ 2.38 | 830.72 $\pm$ 267.34 |

The dispense pressures needed to achieve the ideal mass extremes were interpolated based on HPMC solution viscosity and dispense tip ID ranges. A range of dispense tip IDs and a range of HPMC solution viscosities may be encountered commercially. For example, manufacturers may declare a range of dispense tip IDs between 90 $\mu$m - 130 $\mu$m (0.0035" - 0.0050"). Similarly, viscosities of a 3% w:w F4M-HPMC solution can range between 7,253 cP - 11,520 cP depending on the deviation of the concentration away from being exactly 3% w:w. Table 7 illustrates the dispense mass range for various dispense tip diameters and 3% w:w HPMC solution viscosities.

**Table 7**

| Pressure (psi) needed to dispense the target mass range | | Dispense Tip Diameter | | | |
|---|---|---|---|---|---|
| | | .0035 in (~90 $\mu$m) | 0.0040 in (~100 $\mu$m) | .0045 in (~115 $\mu$m) | .0050 in (~130 $\mu$m) |
| Viscosity | 6,780 cP | 43-52 | 41-51 | 33-42 | 31-39 |
| | 7,880 cP | 47-59 | 45-56 | 37-46 | 35-43 |
| | 10,680 cP | 58-71 | 56-68 | 46-57 | 43-53 |
| | 12,780 cP | 69-74 | 60-72 | 50-71 | 46-56 |

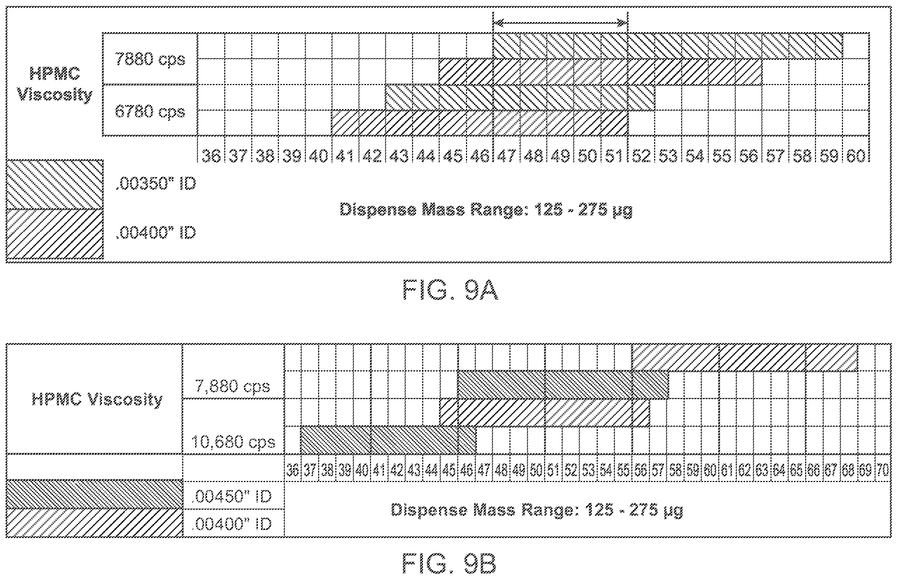

To form recommendations per range, rather than individual values of ID and HPMC viscosity, the pressures that satisfy all ranges was identified. For example, for viscosity range of 6,780 - 7,880 cP and an ID range of 90 - 100 μm, the pressure range is between 47 - 51 psi for a desired dispensed mass of between 125 - 275 μg (see FIG. 9A). High jumps were noted between pressure values for 0.0040" and 0.0045" ID dispense tips and between 7,880 and 10,680 cP HPMC solutions (see FIG. 9B). In this case, no common pressure range existed that would cover the analyzed viscosity and ID ranges.

Thus, values to populate the ranges in between the characterized ID/viscosity combinations were interpolated. Table 8 shows the missing values to be calculated based upon the available values. The dispense pressure recommendations were made for each range. To alleviate wide gaps found between the ranges, the following interpolation values were added: In dispense tip ID domain - 0.00375", 0.00425", and 0.00475"; in HPMC viscosity domain - 9,000 cP, 9,500 cP, and 10,000 cP. Table 9 shows the results upon the interpolation.

**Table 8**

| | | Dispense Tip Diameter | | | | | | |
|---|---|---|---|---|---|---|---|---|
| | | .00350 in | .00375 in | .00400 in | .00425 in | .00450 in | .00475 in | .00500 in |
| Viscosity | 6,700 cp | 43–52 | | 41–51 | | 33–42 | | 31–39 |
| | 7,880 cP | 47–59 | | 45–56 | | 37–46 | | 35–43 |
| | 9,000 cP | | | | | | | |
| | 9,500 cP | | | | | | | |
| | 10,000 cP | | | | | | | |
| | 10,680 cP | 58–71 | | 56–68 | | 46–57 | | 43–53 |
| | 12,780 cP | 69–74 | | 60–72 | | 50–61 | | 46–56 |
| | Dispense Mass Range: 125–275 μg (±10 μg) | | | | | | | |

**Table 9**

| Pressure range to dispense target mass | Dispense Tip Diameter | | | | | |
|---|---|---|---|---|---|---|
| | .00350–.00375 in | .00375–.00400 in | .0040–.00425 in | .00425–.0045 in | .0045–.00475 in | .00475–.0050 in |
| **Viscosity** 6,780–7,880 cP | 47–51 | | 45–46.6 | 41–42 | 37–39 | |
| 7,880–9,000 cP | 50.9–56.8 | 49.9–56 | 50.1–51.0 | 45.7–46 | 41.1–45.2 | 40.8–43 |
| 9,000–9,500 cP | 52.9–62.6 | 51.7–61.7 | 51.9–56.4 | 47.3–51.1 | 42.6–50.2 | 42.2–47.8 |
| 9,500–10,000 cP | 55.1–64.6 | 53.6–63.8 | 53.5–58.3 | 48.9–52.9 | 44.1–51.9 | 43.6–49.4 |
| 10,000–10,680 cP | 58–66.4 | 56.3–65.5 | 56–66.0 | 51.1–54.5 | 46–53.5 | 45.3–50.8 |
| 10,680–12,780 cP | 69–68.8 | 63.8–68 | 60–62.6 | 54.7–57 | 50–53 | |

The ranges illustrate the most likely scenarios where the dispense tip ID is 0.00375 in - 0.0045 in (95 - 115 $\mu$m) and the HPMC viscosity is 9,000 - 10,000 cP, the dispense pressure range of 50 - 55 psi will produce an adequate plug in an injection needle of an applicator.

EXAMPLE 4 - CLINICAL DATA SHOWING HPMC RETENTION PLUG PREVENTED INADVERTENT RELEASE OF IMPLANT FROM APPLICATOR

A 3% w:w F4M-HPMC solution was deposited within 28 gauge needle bores of implant applicators to form an HPMC plug as a retention system for bimatoprost SR implants. The applicators were used to administer the implants into patients. Of 2,699 total administrations performed, 40 applicator failures occurred, but only 2 failures were attributed to the polymer retention system failing to maintain the implant within the needle. This represents a 0.07% failure rate attributed to the implant retention system for an over 99.9% retention rate of the implants within the needles.

OZURDEX® is a dexamethasone implant for the treatment of diabetic macular edema, macular edema, and uveitis. The implant is delivered to the vitreous using 22 gauge needle applicator. The needle has no plug retention system, but instead includes a bead of silicone extending into the bore just distal to the implant through the wall of the needle. The retention of applicators incorporating the HPMC plug was compared to the retention of the OZURDEX® applicator having no HPMC plug. The HPMC plug failed in just 29 cases compared to 152 retention failures in OZURDEX® over the same time period. The HPMC plug is an effective system with a very high rate of implant retention.

HPMC has been used in combination with polystyrene microbeads to achieve higher IOP elevations in mouse models of glaucoma (Liu and Ding "Establishment of an experimental glaucoma animal model: A comparison of microbead injection with or without hydroxypropyl methylcellulose" Experimental and Therap. Med. 14:1953-1960, 2017). Pleasingly, the retention plug formed of 3% w:w F4M-HPMC solution for retaining the Bimatoprost SR implants and OZURDEX® implants did not cause any detectable adverse events when used for this purpose.

EXAMPLE 5 - EFFECT OF SILICONIZATION ON HPMC PLUG ADHESION WITHIN THE NEEDLE

Needle siliconization is a process for hypodermic needles to reduce the friction between the metal of the needle and tissues being penetrated A thin layer of silicone can be used to coat the tip of the needle to enhance lubrication and reduce friction. Needles can be siliconized according to different techniques. The effect of siliconization technique on HPMC plug formation and adhesion was studied. The HPMC plug was formed from 3% w:w F4M-HPMC and 350 $\mu$g dispensed mass into the bore of a 22 gauge needle. Needle tips were lubricated by dipping in silicone oil or spraying-on the silicone oil so that the bevel of the needle was oriented away from the spray nozzle. The needles that are dipped in silicone have their outside surfaces coated including the needle bevel and opening so that the silicone enters the needle bore and coats inside surfaces of the needle as well. Needles that are spray-coated with silicone avoid focuses the silicone to the outside surface while permitting only a small amount of silicone oil to settle on the bevel and in the opening of the lumen. The siliconized needles were compared to fully non-siliconized needles having no silicone on inside or outside surfaces.

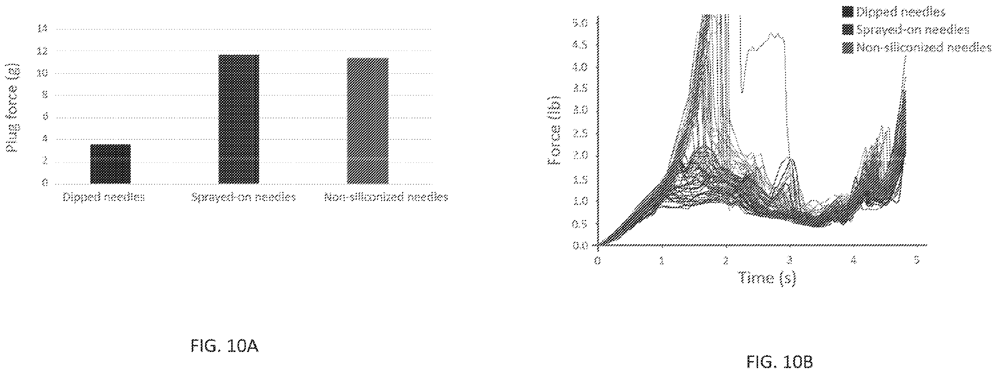

Plug push-out force (POF) was measured using a texture analyzer and steel gauge pin to assess the amount of adhesion achieved by the HPMC plug. The dipped needles with inside and outside surfaces siliconized showed the weakest adhesion compared to the needles having the outside surface siliconized by spray-coating and fully non-siliconized needles (see FIG. 10A). The needle assembly of the dipped siliconized needles failed to retain the implant in 24 of 30 (a failure rate of 80%) whereas all of the sprayed-on siliconized needles assembled retained the implants without issues (100%). All non-siliconized needles assembled and retained the implants (100%). Actuation force is the amount of force measured to press the actuator button on the device to push out the implant through the plug positioned inside the needle bore. Each of the siliconized needle groups was similar in the measured actuation force (FIG. 10B). The non-siliconized needle group, in contrast, showed a strong adhesion between the HPMC plug and the needle bore that resulted in a significant increase in actuation force needed to eject the implant.

The results show that siliconized needles in which the outside surface is spray-coated reducing the amount of silicone oil settling within the bevel had improved HPMC plug adhesion compared to siliconized needles where both outside and inside surfaces were fully coated with silicone and without negatively impacting usability of the device. The siliconized needles having spray-coated outside surfaces did not significantly impact the actuation force needed to deploy the implant as the fully non-siliconized needles. The force required to expel the HPMC plug ahead of the implant is much lower where only a small amount of silicone is present in the bevel opening compared to non-siliconized needles.

EXAMPLE 6 - EFFECT OF HPMC PLUG AND PUSH ROD LENGTH ON IMPLANT DEPLOYMENT

Previous studies found retinal injury can occur following intravitreal injection of a dexamethasone implant in vitrectomized eyes (Lee et al. Int. J. Ophthalmol. 2017; 10(6):1019-1020). Accidental injection of the implant into retinal tissue is rare because implants generally settle on the inferior vitreous cavity even in vitrectomized eye. Vitreous humor is a transparent, gelatinous tissue that is highly viscous in nature due to the presence of collagen fibers, hyaluronin, and opticin. The viscosity can be in a range of 300-2,000 centipoise (cP) and generally provides resistance against projectile velocity of an intraocular implanted injected into the vitreous. Panjaphongse et al. (J Ocul Pharmacol Ther. 2015;31(3):174-178.) reported the OZURDEX® implant can travel 15 mm when injected into vitrectomized eyes.

The HPMC plug was analyzed to assess whether the presence of the plug dampens the ejection speed and provides for a safer intravitreous injection. The kinematics of the implants ejected through plugs formed of different dispensed masses of HPMC was analyzed. The actuation force and ejection distance of implants deployed from spray-siliconized 22G needles having 3% w:w HPMC dispensed mass of 300 $\mu$g, 650 $\mu$g, and 1000 $\mu$g were tested. The dispensed mass 300 $\mu$g had an actuation force of 8.308±1.092 (mean±SD), dispensed mass 650 $\mu$g had an actuation force of 9.085±1.115 (mean±SD) and dispensed mass 1000 $\mu$g had an actuation force of 9.563±0.988 (mean±SD). The actuation force to deploy the implant significantly increased as the dispensed mass increased, but all samples had an actuation force that was less than or equal to a maximum upper limit of 22N. Further, ejection distance for each sample configuration was not significantly different. The HPMC plugs formed from 300-1000 $\mu$g in 22G needles were viable retention systems that did not impact performance in terms of actuation force and ejection distance. It is notable that HPMC dispense mass did not affect ejection distance although actuation force was affected by HPMC dispense mass.

Push rod length was tested to assess its impact on ejection performance through a 22 gauge needle incorporating an implant retention system. Group 1 applicators (n=29) had push rods that were 27.64 mm long such that the distal end of the push rod extended past the distal-most tip of the needle upon complete actuation. Group 2 applicators (n=29) had push rods that were 26.64 mm long and Group 3 applicators (n=29) had push rods that were 25.64 mm long. The push rods of Group 2 and Group 3 applicators did not extend past the distal-most tip of the needle upon complete actuation. Group 1 incorporated an outer silicone sleeve implant retention system whereas Group 2 and Group 3 applicators included an HPMC plug inside the needle bore as the implant retention system. The HPMC plug was formed using 3% w:w F4M-HPMC and a dispensed mass of 650 $\mu$g ± 200 $\mu$g. Needles in Group 1 applicators were siliconized by dipping and needles in Groups 2 and 3 were siliconized by spraying-coating the outside surface as described elsewhere herein.

The applicators of each of the three groups were tested *in vitro* by manually actuating the button to eject an implant into a balanced salt solution (BSS) as the medium. A high speed camera was used to capture the implant trajectory at 2,800 frames/second. The initial speed, final speed, and ejection distance traveled by the implant within the BSS were measured for each group. Initial speed is the speed of the implant captured in the first frame at the time the implant separates from the needle after actuation. Final speed is the speed of the implant captured in the last frame. Ejection distance is the distance traveled by the implant from the distal tip of the needle into the BSS until the implant reached a "safe" speed of 10 mm/second. An implant traveling at 10 mm/second sinks in the BSS under the force of gravity and is considered to be a safe final speed that is unlikely to cause any damage to a retina. Once the implants reach this safe speed, the distance covered following ejection from the needle, referred to as the ejection distance, was measured.

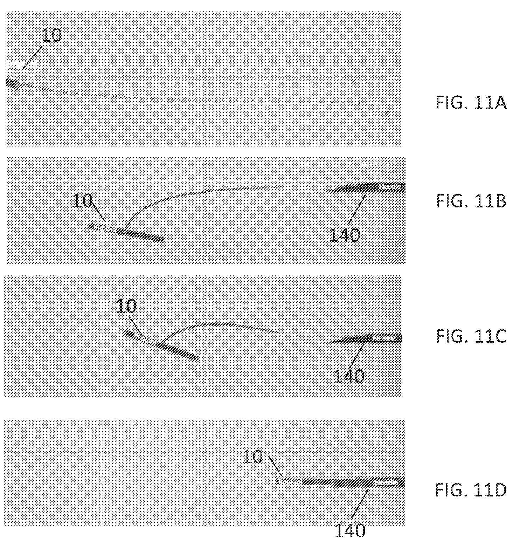

FIGs. 11A-11D show typical ejection patterns observed for each group tested. Group 1 applicators ejected the implants 10 along a generally straight trajectory (see FIG. 11A). Group 2 and Group 3 applicators ejected the implants 10 along a curved trajectory relative to a longitudinal axis of the needle 140 (FIGs. 11B and 11C, respectively). The curved trajectory is indicative of the sinking of the implant in the BSS due to the force of gravity on the implant as it slows to the safe speed. FIG. 11D shows an example of an implant 10 that failed to detach from the needle 140. Implants that fail to detach from the distal end of the needle is generally an undesirable result as the implant can potentially cause tissue damage as the needle is withdrawn from the eye.

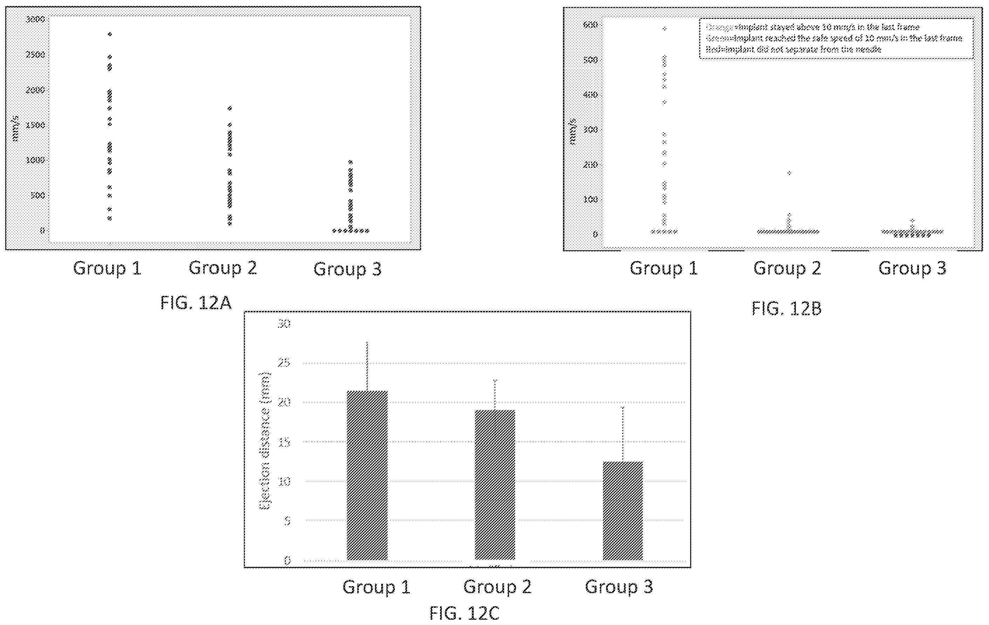

FIGs. 12A-12C shows for each applicator group showing average initial speed, average final speed, and average ejection distance, respectively. Table 10 below provides the data (mean ± SD). The implants ejected from Group 1 applicators having the longest push rod length and the silicone sleeve retention system had a high average initial speed of 1,434.6±670.3 mm/s. Only 5 of the 29 implants (17%) decelerated to the safe speed of 10 mm/s and 24 implants (83%) stayed above the safe speed of 10 mm/s by the final frame. The average final speed was 230.0±188.6 mm/s. The average ejection distance for the 5 implants that reached the safe speed was 21.4±6.25 mm. All implants from Group 1 applicators successfully detached from the needle. The implants ejected from Group 2 applicators having the 1 mm shorter push rods and HPMC plugs had a slower average initial speed of 756±470.2 mm/s, which is nearly twice as slow as implanted ejection from Group 1 applicators. Of the 29 implants in Group 2 applicators, 17 implants (59%) decelerated to the safe speed of 10 mm/s by the final frame and 12 implants (41%) stayed above 10 mm/s in the final frame. The average final speed was 23.0±32.4 mm/s, which was ten times slower than the Group 1 applicators. The average ejection distance for the 17 implants that reached the safe speed was 19.0±3.78 mm, which was similar to Group 1. Also like Group 1, there were no instances of the implant failing to detach from the needle in the Group 2 applicators. The implants ejected from Group 3 applicators having the 2 mm shorter push rods and HPMC plugs had a slower average initial speed of 422.2 ±297.4 mm/s (excluding cases that failed to detach from the needle). The average initial speed was even slower including those implants that failed to detach from the needle and was 316.6 ± 316.5 mm/s. Of the 29 implants in Group 3 applicators, 15 implants (52%) decelerated to the safe speed of 10 mm/s by the final frame and 6 implants (22%) stayed above the safe speed of 10 mm/s in the final frame. Seven implants (24%) failed to detach from the needle in Group 3 applicators. The average final speed was 10.5±9.4 mm/s including the implants that failed to detach and 14.0 ± 8.2 mm/s excluding the implants that failed to detach. The average ejection distance for the 15 implants that reached the safe speed was 12.5 ± 6.91 mm.

**Table 10**

| Applicator Group | Average Initial Speed (mm/s) | # decelerated by final frame to 10 mm/s | Average Final Speed (mm/s) | Ejection Distance (mm) |
| --- | --- | --- | --- | --- |
| 1 | 1,434.6 ± 670.3 | 5 (17%) | 230.0 ± 188.6 | 21.4 ± 6.25 |
| 2 | 756 ± 470.2 | 17 (59%) | 23.0 ± 32.4 | 19.0 ± 3.78 |
| 3 | 422.2 ± 297.4 | 15 (52%) | 14.0 ± 8.2 | 12.5 ± 6.91 |

The applicators with the shorter push rod lengths and having the HPMC plugs present in the needle bore (Groups 2 and 3) had significantly slower initial speeds and final speeds compared to applicators with the longer push rods and no HPMC plug present in the bore (Group 1). The applicators with the shortest push rod length (Group 3) also had a significantly shorter ejection distance compared to the other two groups. However, these applicators slowed down to the point that there was an increased incidence of implants failing to detach compared to the other applicator groups having longer push rods, which never failed to detach. Neither Group 2 nor Group 3 push rods extended past the distal-most tip of the needle. However, the implants in Group 3 can get wedged within the narrow part of the needle bevel suggesting the push rod length was too short to reliably deploy the implant. The applicators of Group 2 incorporating an HPMC plug and shortened push rod length slowed the initial and final speeds while achieving a sufficient ejection distance without issues of the implant failing to detach. Thus, the applicators in Group 2 provided a reliable and safe implant delivery with lower likelihood of retinal strikes. A shortened push rod can have a length that is less than 2 mm shorter than a length of the standard push rod. The shortened push rod can have a length that is about 1 mm shorter than a length of the standard push rod or a length that is greater than about 26 mm and less than about 27 mm.

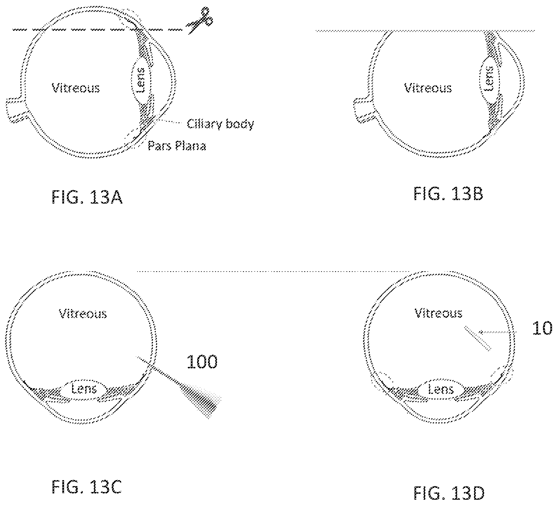

The slower ejection speeds of the Group 2 applicators were assessed for their ability to position the implant in the eye using the Miyake-Apple technique modified from Davis *et al.* (Davis BL, Nilson CD, Mamalis N. Revised Miyake-Apple technique for postmortem eye preparation. J Cataract Refract Surg. 2004 Mar; 30(3):546-9.). The technique involves porcine eye globes prepared so as to visualize the implant in the vitreous cavity after deployment. Eyes were stabilized by suction on a Mastel mount and placed such that the cornea was facing forward. Approximately 1/5 of the globe was cut from the top creating a "window" into the vitreous cavity for visualization (see FIG. 13A). A glass coverslip was then glued over the rim of the sclera (see FIG. 13B). The Group 2 applicators described in Example 6 were used for the injection of the implant into the vitreous cavity (see FIG. 13C) and images capture of the implant position within the vitreous (see FIG. 13D).

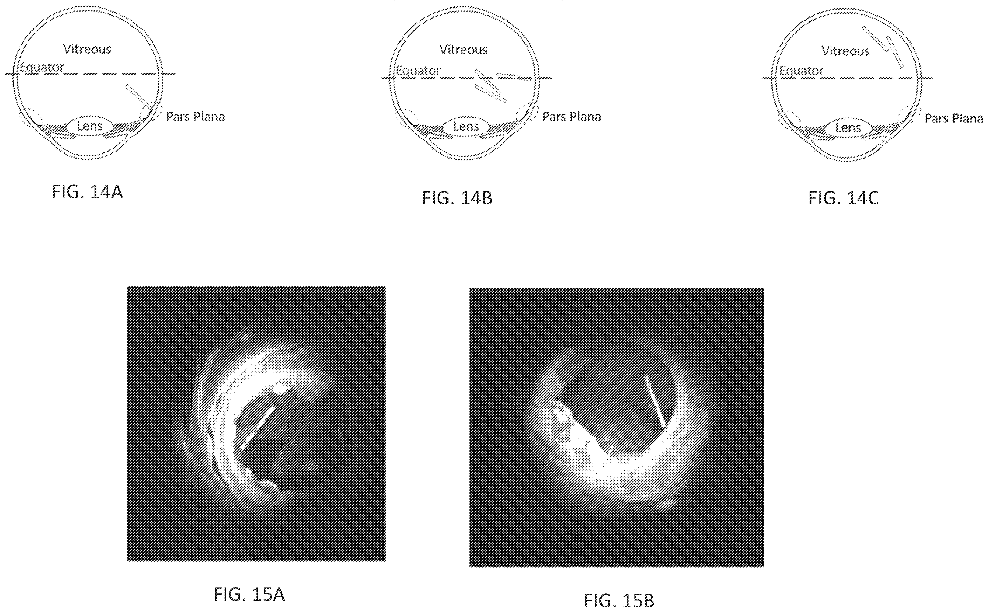

The intraocular position of the implant discerned from the images captured during and after actuation were evaluated based on the methodology described by Sudhalkar *et al.* (Sudhalkar A, Kodjikian L, Chhablani J, Bhojwani D, Vasavada A. Intraocular dexamethasone implant position in situ and ocular hypertension. Retina. 2018 Dec; 38(12):2343-2349). Position 1 (P1) was defined as the implant lying either flush to (radial to the *pars plana/plicata*) or perpendicular to the ciliary region with at least a part of the implant in contact with the ciliary region (see FIG. 14A). Position 2 (P2) was defined as the implant visible in the vitreous cavity anterior to the equator of the eye globe but with no part of it in contact with the ciliary body (see FIG. 14B). Position 3 (P3) was defined as the presence of the implant posterior to the equator (see FIG. 14C). FIG. 15A shows a representative image of an implant positioned in the P1 position in the vitreous chamber in contact with the pars plana. FIG. 15B shows a representative image of an implant positioned in the P2 position in the vitreous chamber anterior to or on the equator. The Group 2 applicators (n = 22) having a 1 mm shortened push rod and the HPMC plug positioned in the bore was compared *ex vivo* with the Group 1 applicators (n = 11) having a standard push rod length and no HPMC plug to evaluate the implant position in the vitreous cavity after deployment in explanted porcine eyes. Each of Group 1 and Group 2 applicators performed similarly in that the implant landed in either the P1 or P2 positions, away from the retina. Group 1 applicators had 6 implants land in the P1 position and 5 implants land in the P2 position. Group 2 applicators had 15 implants land in the P1 position and 7 implants land in the P2 position. None of the implants in either group landed in the P3 position closer to the retina.

A small user experience study was conducted to compare Group 1 and Group 2 applicators *ex vivo.* Users were ophthalmologists who performed the injection procedure in a direct side-by-side comparison with each of the applicator types. The users performed in the injection procedure per product requirements in explanted pig eyes using 20 samples per applicator type. Usability scores were recorded from worse to best on a 1 - 4 continuous sliding scale. The data is shown in Table 11 below. The applicators in Group 2 performed better in terms of the usability of the actuator button press. Group 1 applicators incorporated a silicone sleeve on an outer surface of the needle that acted as a retention system. Group 2 did not have this silicone sleeve, but instead incorporated an integrated elongated needle hub having an ABS distal extension. The applicators of Group 2 having the modified sleeve surface did not adversely affect the tissue contact compared to applicators of Group 1 having the silicone sleeves. Needle glide into the tissue was perceived to be better in the applicators of Group 2.

**Table 11**

| Performance Characteristic | System Usability Score (average) | |
|---|---|---|
| | Group 1 applicator | Group 2 applicator |
| Button Actuation: 1 = poor performance; 4 = very good performance | | |
| • Button force | 3.3 | 3.8 |
| • Button control | 3.2 | 3.8 |
| Needle and Sleeve: 1 = scleral damage likely; 4 = scleral damage unlikely | | |
| • Sleeve surface | 4.0 | 3.8 |
| • Needle gliding | 3.4 | 4.0 |

EXAMPLE 7 - CLINICAL STUDY COMPARING SUBJECT APPLICATOR TO

COMMERCIAL APPLICATION

A clinical study with an example apparatus as described herein.

**Objective.** The objective of the study was to demonstrate that the subject applicator described herein delivers the implant to the vitreous cavity and is suitable for commercial use.

**Study design.** The study was multi-center and open-label. There were two treatment arms with active comparator: applicator described herein (having a 3% w:w HPMC retention plug with plunger length of 1.049 ± 0.005in) versus currently approved commercial applicator (without the HPMC retention plug). There were approximately 54 patients with diabetic macular edema (DME) or macular edema due to retinal vein occlusion (central or branch; CRVO or BRVO). Patients were randomized in a 2:1 ratio to receive the OZURDEX® (0.7 mg dexamethasone implant) using either the applicator described herein (test) or the currently approved commercial applicator (comparator), respectively, in the study eye. The administration was a single dose administration, and the treatment period was a 7-day treatment period, where the implant was administered (using the respective applicator, test or comparator) at day 1 to the study eye and followed until day 7.

**Study endpoints.** No efficacy or safety endpoints evaluated since OZURDEX® is an FDA-approved drug, and its efficacy and safety is already well-established. Safety variables were evaluated (vital signs, adverse events, and ocular parameters as determined through assessments of BCVA, biomicroscopic slit-lamp examination, ophthalmoscopy, and intraocular pressure) along with applicator performance of both applicators (test and comparator) via treatment administration assessment.

**Results.** Performance of the applicators (test and comparator) were assessed via a treatment administration assessment completed by investigator after administration (Day 1).

The summary of the performances of the applications is shown in table 12 below.

**Table 12**

| Parameter | Test applicator group | Comparator applicator group |
|---|---|---|
| | N=36 n(%) | N=18 n(%) |
| **Implant administration performed with the initial assigned kit** | | |
| Yes | 36 (100.0) | 18 (100.0) |
| No | 0 (0.0) | 0 (0.0) |
| **Applicator dispensed the implant in the vitreous cavity** | | |
| Yes | 36 (100.0) | 18 (100.0) |
| No | 0 (0.0) | 0 (0.0) |
| **Applicator performed as intended** | | |
| Yes | 36 (100.0) | 18 (100.0) |
| No | 0 (0.0) | 0 (0.0) |

No product complaints were reported during the study.

Ocular treatment emergent adverse events (TEAEs) reported during the study are summarized in table 13 below.

**Table 13**

| | Test applicator group | | Comparator applicator group | |
|---|---|---|---|---|
| | Study Eye N=36 n (%) | Fellow Eye* N=36 n (%) | Study Eye N=18 n (%) | Fellow Eye* N=18 n (%) |
| **Any ocular adverse event** | 12 (33.3) | 0 (0.0) | 7 (38.9) | 0 (0.0) |
| **Conjunctival hemorrhage** | 10 (27.8) | 0 (0.0) | 7 (38.9) | 0 (0.0) |
| **Eye pain** | 1 (2.8) | 0 (0.0) | 0 (0.0) | 0 (0.0) |
| **Lacrimation increased** | 1 (2.8) | 0 (0.0) | 0 (0.0) | 0 (0.0) |
| **Photophobia** | 1 (2.8) | 0 (0.0) | 0 (0.0) | 0 (0.0) |
| **Injection site pain** | 1 (2.8) | 0 (0.0) | 0 (0.0) | 0 (0.0) |

(continued)

| | Test applicator group | | Comparator applicator group | |
|---|---|---|---|---|
| | Study Eye N=36 n (%) | Fellow Eye* N=36 n (%) | Study Eye N=18 n (%) | Fellow Eye* N=18 n (%) |
| **Intraocular pressure increased** | 2 (5.6) | 0 (0.0) | 0 (0.0) | 0 (0.0) |
| | | | | * Fellow eye was allowed standard of care |

Ocular TEAEs possibly related to study treatment administration in the study eye are summarized in Table 14 below.

**Table 14**

| | Test applicator group N=36 n(%) | Comparator applicator group N=18 n(%) |
|---|---|---|
| **Any adverse event possibly related to study treatment administration in the study eye** | 10 (27.8) | 6 (33.3) |
| **Conjunctival hemorrhage** | 8 (22.2) | 6 (33.3) |
| **Eye pain** | 1 (2.8) | 0 (0.0) |
| **Injection site pain** | 1 (2.8) | 0 (0.0) |
| **Intraocular pressure increased** | 2 (5.6) | 0 (0.0) |

The AEs observed were similar to those reported in the OZURDEX® label.

**Conclusion.** All applicators used in the study performed as intended. No issues with the usability of the applicator were reported. Safety profile of the applicator looks similar to safety information in the OZURDEX® Label.

In aspects, description is made with reference to the figures. However, certain aspects may be practiced without one or more of these specific details, or in combination with other known methods and configurations. In the description, numerous specific details are set forth, such as specific configurations, dimensions, and processes, in order to provide a thorough understanding of the implementations. In other instances, well-known processes and manufacturing techniques have not been described in particular detain in order to not unnecessarily obscure the description. Reference throughout this specification to "one embodiment," "an embodiment," "an aspect," "one aspect," "one implementation, "an implementation," or the like, means that a particular feature, structure, configuration, or characteristic described is included in at least one embodiment, aspect, or implementation. Thus, the appearance of the phrase "one embodiment," "an embodiment," "one aspect," "an aspect," "one implementation, "an implementation," or the like, in various placed throughout this specification are not necessarily referring to the same embodiment, aspect, or implementation. Furthermore, the particular features, structures, configurations, or characteristics may be combined in any suitable manner in one or more implementations.

The use of relative terms throughout the description may denote a relative position or direction or orientation and is not intended to be limiting. For example, "distal" may indicate a first direction away from a reference point. Similarly, "proximal" may indicate a location in a second direction opposite to the first direction. Use of the terms "front," "side," and "back" as well as "anterior," "posterior," "caudal," "cephalad" and the like or used to establish relative frames of reference, and are not intended to limit the use or orientation of any of the devices described herein in the various implementations.

The word "about" means a range of values including the specified value, which a person of ordinary skill in the art would consider reasonably similar to the specified value. In embodiments, about means within a standard deviation using measurements generally acceptable in the art. In embodiments, about means a range extending to +/- 10% of the specified value. In embodiments, about includes the specified value.

While this specification contains many specifics, these should not be construed as limitations on the scope of what is claimed or of what may be claimed, but rather as descriptions of features specific to particular embodiments. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or a variation of a sub-combination. Similarly, while operations are depicted in the drawings in a particular order, this should not be understood as requiring that such operations be performed in the particular order shown or in sequential order, or that all illustrated operations be performed, to achieve desirable results. Only a few examples, embodiments, aspects, and implementations are disclosed. Variations, modifications and enhancements to the described examples and implementations and other implementations may be made based on what is disclosed.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together."

Use of the term "based on," above and in the claims is intended to mean, "based at least in part on," such that an unrecited feature or element is also permissible.

Throughout this specification reference is made to publications such as US and foreign patent applications, journal articles, book chapters, and others. All such publications are expressly referred to, including supplemental/supporting information sections published with the corresponding references, for all purposes unless otherwise indicated. To the extent that any recitations in the references conflict with any recitations herein, the recitations herein will control.

The foregoing descriptions details methods that can be employed to treat various ocular conditions, and represents the best mode contemplated. It should not be construed as limiting the overall scope hereof; rather, the ambit of the present disclosure is to be governed only by the lawful construction of the appended claims.

**Claims**

1. A system for treating an eye of a patient in need, the system comprising:
   a delivery device (100) comprising:

   a housing (120) sized to be held by an operator and comprising an actuator (150) and a distal nose cone (130);
   a needle (140) coupled to and extending distal from the nose cone (130), the needle (140) having a lumen and a beveled distal end defining a distal opening having a sharpened tip (141) and heel proximal the distal opening; and
   a retention plug (180) positioned within and spanning the lumen of the needle (140) proximal to the heel, the retention plug (180) formed from a flowable retainer solution of hydroxypropyl methylcellulose (HPMC) in water having a concentration that is greater than 2.5% w:w and less than about 4% w:w; and
   an intraocular implant (10) positioned within the lumen of the needle (140) proximal to the retention plug (180).

2. The system of claim 1, wherein the delivery device (100) further comprises a push rod operatively coupled to the actuator (150) via a linkage to move the push rod through the lumen and push the implant (10) out from the needle (140).

3. The system of claim 2, wherein the push rod has a length relative to a length of the needle (140) sufficient for a distal end of the push rod to advance past the sharpened tip (141) of the needle (140) upon deployment of the implant (10) using the actuator (150).

4. The system of claim 2, wherein the push rod has a shortened length relative to a length of the needle (140) to avoid a distal end of the push rod advancing past the sharpened tip (141) of the needle (140) upon deployment of the implant (10) using the actuator (150),

   wherein preferably, the shortened length of the push rod slows an ejection speed of the implant (10) from the lumen upon deployment using the actuator (150),
   wherein more preferably the retention plug (180) further slows the ejection speed of the implant (10).

5. The system of any one of claims 1 to 4, wherein the retention plug (180) prevents inadvertent release of the implant (10) from the lumen prior to actuation of the device,

and/or wherein the needle (140) has a siliconized external surface and the lumen is substantially non-siliconized.

6. The system of any one of claims 1 to 5, wherein the retention plug (180) slows an ejection speed of the implant (10) from the lumen upon deployment using the actuator (150),
wherein optionally, the ejection speed is no greater than about 25 mm/second at an ejection distance of about 20 mm away from the lumen.

7. The system of any one of claims 1 to 6, wherein the flowable retainer solution of HPMC in water has a HPMC concentration of 3% w:w,

and/or wherein the HPMC is F4M grade having about 27.0%-30.0% methoxyl substitution and about 4.0% - 7.5% hydroxypropoxyl substitution,
and/or wherein the retainer solution has a viscosity between 6,780 cP and 12,780 cP.

8. The system of any one of claims 1 to 7, wherein the needle (140) is sized between 22 gauge and 30 gauge,

optionally wherein the needle (140) is 22 gauge and the retainer solution is dispensed within the lumen of the needle (140) as a dispensed mass greater than 300 $\mu$g and less than 1000 $\mu$g,
or wherein the needle (140) is 28 gauge and the retainer solution is dispensed within the lumen of the needle (140) as a dispensed mass greater than 100 $\mu$g and less than 300 $\mu$g.

9. The system of any one of claims 1 to 8, wherein the delivery device (100) further comprises a cylindrical, distal extension projecting distal from the nose cone (130) and covering at least a proximal end region of the needle (140), the distal extension defining an exposed working length of the needle (140).

10. The system of claim 9, wherein the distal extension has an outer diameter that is greater than a maximum outer diameter of the exposed length of the needle (140), wherein optionally the outer diameter of the distal extension is sized to remain outside the eye and provide a depth stop for the exposed length of the needle (140) sized to be inserted within an eye.

11. The system of claim 9, wherein the distal extension has a length that is 8 mm to 15 mm and the exposed working length of the needle (140) is 5.5 mm to 6.5 mm.

12. The system of claim 9, wherein the distal extension and the nose cone (130) comprises a plastic material and the needle (140) comprises a metal material, wherein optionally, the plastic material is acrylonitrile-butadiene styrene and the metal comprises stainless steel.

13. The system of any one of claims 1 to 12, wherein the intraocular implant (10) comprises a therapeutic and a biodegradable polymer matrix comprising at least one biodegradable polymer, preferably wherein the therapeutic comprises dexamethasone or Bimatoprost,
and optionally, wherein the therapeutic ocular implant (10) is a dexamethasone implant for the treatment of diabetic macular edema.

14. A method of retaining an implant (10) in an implant administration device, the method comprising:

formulating a flowable retainer solution of hydroxypropyl methylcellulose (HPMC);
dispensing a dispensed mass of the retainer solution into a lumen of a needle (140) to form a retention plug (180) that is contained within and spanning the lumen of the needle (140) proximal to a distal opening from the needle (140);
positioning an intraocular implant (10) within the lumen of the needle (140) proximal to the retention plug (180); and
positioning a shortened push rod proximal to the implant (10), the push rod operatively coupled to an actuator (150) of the implant administration device,
wherein the retention plug (180) prevents inadvertent release of the implant (10) from the lumen prior to actuation of the implant administration device and works in conjunction with the shortened push rod to slow the ejection speed of the implant (10) from the lumen upon deployment in the eye using the actuator (150); wherein the needle (140) is 22 Gauge or smaller and the lumen is substantially non-siliconized;
wherein the needle (140) has a siliconized external surface;

wherein the ejection speed is no greater than about 25 mm/second at an ejection distance of about 20 mm away from the lumen; and

wherein the retainer solution has a concentration that is greater than 2.5% w:w and less than about 4% w:w.

**Patentansprüche**

1.  System zur Behandlung eines Auges eines behandlungsbedürftigen Patienten, umfassend:
    eine Freisetzungsvorrichtung (100), die Folgendes umfasst:

    ein Gehäuse (120), das so dimensioniert ist, dass es von einem Bediener gehalten werden kann, und einen Betätiger (150) und ein distales Nasenstück (130) umfasst;
    eine Nadel (140), die mit dem Nasenstück (130) gekoppelt ist und sich distal davon erstreckt, wobei die Nadel (140) ein Lumen und ein abgeschrägtes distales Ende aufweist, das eine distale Öffnung mit einer geschärften Schliffspitze (141) und einer proximal der distalen Öffnung liegenden Schliffferse definiert; und
    einen Rückhaltestopfen (180), der innerhalb des Lumens der Nadel (140) proximal von der Schliffferse das Lumen überbrückend angeordnet ist, wobei der Rückhaltestopfen (180) aus einer fließfähigen Retentionslösung von Hydroxypropylmethylcellulose (HPMC) in Wasser gebildet ist, die eine Konzentration aufweist, die größer als 2,5 Gew.-% (w/w) und weniger als etwa 4 Gew.-% (w/w) ist; und
    ein intraokulares Implantat (10), das innerhalb des Lumens der Nadel (140) proximal von dem Rückhaltestopfen (180) angeordnet ist.

2.  System nach Anspruch 1, wobei die Freisetzungsvorrichtung (100) weiter eine Schubstange umfasst, die über ein Gestänge wirkverbunden mit dem Betätiger (150) ist, um die Schubstange durch das Lumen zu bewegen und das Implantat (10) aus der Nadel (140) herauszudrücken.

3.  System nach Anspruch 2, wobei die Schubstange eine Länge relativ zu einer Länge der Nadel (140) aufweist, die ausreichend ist, damit ein distales Ende der Schubstange beim Ausstoßen des Implantats (10) mittels des Betätigers (150) über die Schliffspitze (141) der Nadel (140) hinaus vorrückt.

4.  System nach Anspruch 2, wobei die Schubstange eine verkürzte Länge relativ zu einer Länge der Nadel (140) aufweist, um zu vermeiden, dass ein distales Ende der Schubstange beim Ausstoßen des Implantats (10) mittels des Betätigers (150) über die Schliffspitze (141) der Nadel (140) hinaus vorrückt,

    wobei vorzugsweise die verkürzte Länge der Schubstange die Ausstoßgeschwindigkeit des Implantats (10) aus dem Lumen beim Ausstoßen mittels des Betätigers (150) verlangsamt,
    wobei besonders bevorzugt der Rückhaltestopfen (180) die Ausstoßgeschwindigkeit des Implantats (10) weiter verlangsamt.

5.  System nach einem der Ansprüche 1 bis 4, wobei der Rückhaltestopfen (180) ein unbeabsichtigtes Freisetzen des Implantats (10) aus dem Lumen vor der Betätigung der Verabreichungsvorrichtung verhindert,
    und/oder wobei die Nadel (140) eine silikonisierte Außenoberfläche aufweist und das Lumen im Wesentlichen nicht silikonisiert ist.

6.  System nach einem der Ansprüche 1 bis 5, wobei der Rückhaltestopfen (180) die Ausstoßgeschwindigkeit des Implantats (10) aus dem Lumen beim Ausstoßen mittels des Betätigers (150) verlangsamt,
    wobei wahlweise die Ausstoßgeschwindigkeit nicht größer als etwa 25 mm/s bei einer Auswurfdistanz von etwa 20 mm vom Lumen ist.

7.  System nach einem der Ansprüche 1 bis 6, wobei die fließfähige Retentionslösung aus HPMC in Wasser eine HPMC-Konzentration von 3 Gew.-% (w/w) aufweist,

    und/oder wobei das HPMC der Qualität F4M ist mit einer Methoxylsubstitution von etwa 27,0 % - 30,0 % und einer Hydroxypropoxylsubstitution von etwa 4,0 % - 7,5 %,
    und/oder wobei die Retentionslösung eine Viskosität zwischen 6.780 und 12.780 cP aufweist.

8.  System nach einem der Ansprüche 1 bis 7, wobei die Nadel (140) eine Größe zwischen 22 Gauge und 30 Gauge aufweist,

wahlweise wobei die Nadel (140) eine Größe von 22 Gauge aufweist, und die Retentionslösung innerhalb des Lumens der Nadel (140) als abgegebene Masse von größer als 300 μg und kleiner als 1.000 μg abgegeben wird, oder wobei die Nadel (140) eine Größe von 28 Gauge aufweist, und die Retentionslösung innerhalb des Lumens der Nadel (140) als abgegebene Masse von größer als 100 μg und kleiner als 300 μg abgegeben wird.

9. System nach einem der Ansprüche 1 bis 8, wobei die Freisetzungsvorrichtung (100) weiter einen zylindrischen, distalen Fortsatz umfasst, der distal vom Nasenstück (130) vorspringt und zumindest einen proximalen Endbereich der Nadel (140) abdeckt, wobei der distale Fortsatz eine freiliegende Arbeitslänge der Nadel (140) definiert.

10. System nach Anspruch 9, wobei der distale Fortsatz einen Außendurchmesser aufweist, der größer ist als ein maximaler Außendurchmesser der freiliegenden Arbeitslänge der Nadel (140), wobei wahlweise der Außendurchmesser des distalen Fortsatzes so bemessen ist, dass er außerhalb des Auges verbleibt und einen Tiefenanschlag für die freiliegende Arbeitslänge der Nadel (140), die zum Einführen in ein Auge bemessen ist, bereitstellt.

11. System nach Anspruch 9, wobei der distale Fortsatz eine Länge von 8 mm bis 15 mm aufweist und die freiliegende Arbeitslänge der Nadel (140) 5,5 mm bis 6,5 mm beträgt.

12. System nach Anspruch 9, wobei der distale Fortsatz und das Nasenstück (130) ein Kunststoffmaterial umfassen und die Nadel (140) ein Metallmaterial umfasst, wobei wahlweise das Kunststoffmaterial Acrylnitril-Butadien-Styrol (ABS) ist und das Metall Edelstahl umfasst.

13. System nach einem der Ansprüche 1 bis 12, wobei das intraokulare Implantat (10) einen therapeutischen Wirkstoff und eine bioabbaubare Polymermatrix umfasst, die mindestens ein bioabbaubares Polymer umfasst, wobei vorzugsweise der therapeutische Wirkstoff Dexamethason oder Bimatoprost umfasst,
und wahlweise, wobei das therapeutische intraokulare Implantat (10) ein Dexamethason-Implantat zur Behandlung eines diabetischen Makulaödems ist.

14. Verfahren zur Zurückhaltung eines Implantats (10) in einer Implantat-Verabreichungsvorrichtung, umfassend:

Formulieren einer fließfähigen Retentionslösung aus Hydroxypropylmethylcellulose (HPMC);
Abgeben einer abgegebenen Masse der Retentionslösung in ein Lumen einer Nadel (140), um einen Rückhaltestopfen (180) zu bilden, der innerhalb des Lumens der Nadel (140) enthalten ist und das Lumen proximal von einer distalen Öffnung der Nadel (140) überbrückt;
Anordnen eines intraokularen Implantats (10) innerhalb des Lumens der Nadel (140) proximal von dem Rückhaltestopfen (180); und
Anordnen einer verkürzten Schubstange proximal von dem Implantat (10), wobei die Schubstange wirkverbunden mit einem Betätiger (150) der Implantat-Verabreichungsvorrichtung ist, wobei der Rückhaltestopfen (180) ein unbeabsichtigtes Freisetzen des Implantats (10) aus dem Lumen vor der Betätigung der Implantat-Verabreichungsvorrichtung verhindert und in Verbindung mit der verkürzten Schubstange wirkt, um die Ausstoßgeschwindigkeit des Implantats (10) aus dem Lumen beim Ausstoßen in das Auge mittels des Betätigers (150) zu verlangsamen; wobei die Nadel (140) 22 Gauge oder kleiner ist und das Lumen im Wesentlichen nicht silikonisiert ist;
wobei die Nadel (140) eine silikonisierte Außenoberfläche aufweist;
wobei die Ausstoßgeschwindigkeit nicht größer als etwa 25 mm/s bei einer Auswurfdistanz von etwa 20 mm vom Lumen ist; und
wobei die Retentionslösung eine Konzentration aufweist, die größer als 2,5 Gew.-% (w/w) und weniger als etwa 4 Gew.-% (w/w) ist.

**Revendications**

1. Système de traitement d'un œil d'un patient en ayant besoin, le système comprenant :
un dispositif de mise en place (100) comprenant :

un boîtier (120) dimensionné pour être tenu par un opérateur et comprenant un actionneur (150) et un cône de nez distal (130) ;
une aiguille (140) couplée au cône de nez (130) et s'étendant de manière distale à partir de celui-ci, l'aiguille (140) présentant une lumière et une extrémité distale biseautée définissant une ouverture distale présentant une pointe affûtée (141) et un talon proximal de l'ouverture distale ; et

un bouchon de retenue (180) positionné à l'intérieur de la lumière de l'aiguille (140) et la traversant, de manière proximale au talon, le bouchon de retenue (180) étant formé à partir d'une solution de retenue fluide d'hydroxypropylméthylcellulose (HPMC) dans de l'eau présentant une concentration supérieure à 2,5 % p/p et inférieure à environ 4 % p/p ; et

un implant intraoculaire (10) positionné à l'intérieur de la lumière de l'aiguille (140) de manière proximale au bouchon de retenue (180).

2. Système selon la revendication 1, dans lequel le dispositif de mise en place (100) comprend en outre une tige de poussée couplée fonctionnellement à l'actionneur (150) via une liaison pour déplacer la tige de poussée à travers la lumière et pousser l'implant (10) hors de l'aiguille (140).

3. Système selon la revendication 2, dans lequel la tige de poussée présente, par rapport à une longueur de l'aiguille (140), une longueur suffisante pour qu'une extrémité distale de la tige de poussée avance au-delà de la pointe affûtée (141) de l'aiguille (140) lors du déploiement de l'implant (10) à l'aide de l'actionneur (150).

4. Système selon la revendication 2, dans lequel la tige de poussée présente une longueur raccourcie par rapport à une longueur de l'aiguille (140) pour éviter qu'une extrémité distale de la tige de poussée n'avance au-delà de la pointe affûtée (141) de l'aiguille (140) lors du déploiement de l'implant (10) à l'aide de l'actionneur (150),

dans lequel, préférablement, la longueur raccourcie de la tige de poussée ralentit une vitesse d'éjection de l'implant (10) hors de la lumière lors du déploiement à l'aide de l'actionneur (150),

dans lequel, plus préférablement, le bouchon de retenue (180) ralentit davantage la vitesse d'éjection de l'implant (10).

5. Système selon l'une quelconque des revendications 1 à 4, dans lequel le bouchon de retenue (180) empêche une libération involontaire de l'implant (10) hors de la lumière avant l'actionnement du dispositif,

et/ou dans lequel l'aiguille (140) présente une surface externe siliconée et la lumière est sensiblement non siliconée.

6. Système selon l'une quelconque des revendications 1 à 5, dans lequel le bouchon de retenue (180) ralentit une vitesse d'éjection de l'implant (10) hors de la lumière lors du déploiement de l'implant à l'aide de l'actionneur (150), dans lequel, facultativement, la vitesse d'éjection ne fait pas plus d'environ 25 mm/s à une distance d'éjection d'environ 20 mm de la lumière.

7. Système selon l'une quelconque des revendications 1 à 6, dans lequel la solution de retenue fluide de HPMC dans l'eau présente une concentration de HPMC de 3 % p/p,

et/ou dans lequel le HPMC est de qualité F4M présentant environ 27,0 % à 30,0 % de substitution méthoxyle et environ 4,0 % à 7,5 % de substitution hydroxypropoxyle,

et/ou dans lequel la solution de retenue présente une viscosité comprise entre 6 780 cP et 12 780 cP.

8. Système selon l'une quelconque des revendications 1 à 7, dans lequel l'aiguille (140) présente un calibre compris entre 22 et 30,

facultativement, dans lequel l'aiguille (140) est de calibre 22 et la solution de retenue est distribuée dans la lumière de l'aiguille (140) sous la forme d'une masse distribuée de plus de 300 $\mu$g et de moins de 1 000 $\mu$g, ou dans lequel l'aiguille (140) est de calibre 28 et la solution de retenue est distribuée dans la lumière de l'aiguille (140) sous la forme d'une masse distribuée de plus de 100 $\mu$g et de moins de 300 $\mu$g.

9. Système selon l'une quelconque des revendications 1 à 8, dans lequel le dispositif de mise en place (100) comprend en outre une extension distale cylindrique se projetant de manière distale à partir du cône de nez (130) et recouvrant au moins une région d'extrémité proximale de l'aiguille (140), l'extension distale définissant une longueur utile exposée de l'aiguille (140).

10. Système selon la revendication 9, dans lequel l'extension distale présente un diamètre externe supérieur à un diamètre externe maximal de la longueur exposée de l'aiguille (140), dans lequel, facultativement, le diamètre externe de l'extension distale est dimensionné pour rester à l'extérieur de l'œil et fournir une butée de profondeur pour la longueur exposée de l'aiguille (140) dimensionnée pour être insérée dans un œil.

11. Système selon la revendication 9, dans lequel l'extension distale présente une longueur de 8 mm à 15 mm et la longueur utile exposée de l'aiguille (140) est de 5,5 mm à 6,5 mm.

12. Système selon la revendication 9, dans lequel l'extension distale et le cône de nez (130) comprennent une matière plastique, et l'aiguille (140) comprend une matière métallique, dans lequel facultativement, la matière plastique est de l'acrylonitrile-butadiène-styrène et le métal comprend de l'acier inoxydable.

13. Système selon l'une quelconque des revendications 1 à 12, dans lequel l'implant intraoculaire (10) comprend un agent thérapeutique et une matrice polymère biodégradable comprenant au moins un polymère biodégradable, préférablement dans lequel l'agent thérapeutique comprend de la dexaméthasone ou du Bimatoprost, et facultativement, dans lequel l'implant intraoculaire thérapeutique (10) est un implant de dexaméthasone pour le traitement de l'œdème maculaire diabétique.

14. Procédé de retenue d'un implant (10) dans un dispositif de mise en place d'implant, le procédé comprenant :

la formulation d'une solution de retenue fluide d'hydroxypropylméthylcellulose (HPMC) ;
la distribution d'une masse distribuée de la solution de retenue dans une lumière de l'aiguille (140) pour former un bouchon de retenue (180) qui est contenu dans la lumière de l'aiguille (140) et la traverse de manière proximale à une ouverture distale depuis l'aiguille (140) ;
le positionnement d'un implant intraoculaire (10) à l'intérieur de la lumière de l'aiguille (140) de manière proximale au bouchon de retenue (180) ; et
le positionnement d'une tige de poussée raccourcie de manière proximale à l'implant (10), la tige de poussée étant couplée fonctionnellement à un
actionneur (150) du dispositif de mise en place d'implant,
dans lequel le bouchon de retenue (180) empêche une libération involontaire de l'implant (10) hors de la lumière avant l'actionnement du dispositif de mise en place d'implant et coopère avec la tige de poussée raccourcie pour ralentir la vitesse d'éjection de l'implant (10) hors de la lumière lors du déploiement dans l'œil à l'aide de l'actionneur (150) ; dans lequel l'aiguille (140) est de calibre 22 ou plus petit et la lumière est sensiblement non siliconée ;
dans lequel l'aiguille (140) présente une surface externe siliconée ;
dans lequel la vitesse d'éjection ne fait pas plus d'environ 25 mm/s à une distance d'éjection d'environ 20 mm de la lumière ; et
dans lequel la solution de retenue présente une concentration de plus de 2,5 % p/p et de moins d'environ 4 % p/p.

FIG. 1A

FIG. 1B

FIG. 1C

FIG. 1D

FIG. 1E

FIG. 1F

FIG. 2

FIG. 3A

FIG. 3B

FIG. 4A

FIG. 4B

FIG. 5A

FIG. 5B

FIG. 6A

FIG. 6B

FIG. 7

FIG. 8

HPMC Viscosity

7880 cps

6780 cps

36 37 38 39 40 41 42 43 44 45 46 47 48 49 50 51 52 53 54 55 56 57 58 59 60

.00350" ID

.00400" ID

Dispense Mass Range: 125 - 275 µg

FIG. 9A

HPMC Viscosity

7,880 cps

10,680 cps

36 37 38 39 40 41 42 43 44 45 46 47 48 49 50 51 52 53 54 55 56 57 58 59 60 61 62 63 64 65 66 67 68 69 70

.00450" ID

.00400" ID

Dispense Mass Range: 125 - 275 µg

FIG. 9B

FIG. 10A

FIG. 10B

FIG. 11A

FIG. 11B

FIG. 11C

FIG. 11D

FIG. 12A

FIG. 12B

FIG. 12C

FIG. 13A

FIG. 13B

FIG. 13C

FIG. 13D

FIG. 14A

FIG. 14B

FIG. 14C

FIG. 15A

FIG. 15B

EP 4 366 674 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63219480
- EP 3632482 A1
- US 9039761 B2
- US 8535333 B2
- US 2004054374 A1

**Non-patent literature cited in the description**

- **LIU** ; **DING**. Establishment of an experimental glaucoma animal model: A comparison of microbead injection with or without hydroxypropyl methylcellulose. *Experimental and Therap. Med*, 2017, vol. 14, 1953-1960
- **LEE et al.** *Int. J. Ophthalmol.*, 2017, vol. 10 (6), 1019-1020
- **PANJAPHONGSE et al.** *J Ocul Pharmacol Ther.*, 2015, vol. 31 (3), 174-178
- **DAVIS BL** ; **NILSON CD** ; **MAMALIS N.** Revised Miyake-Apple technique for postmortem eye preparation. *J Cataract Refract Surg.*, March 2004, vol. 30 (3), 546-9
- **SUDHALKAR A** ; **KODJIKIAN L** ; **CHHABLANI J** ; **BHOJWANI D** ; **VASAVADA A**. Intraocular dexamethasone implant position in situ and ocular hypertension. *Retina*, December 2018, vol. 38 (12), 2343-2349